# FASCICULE DE BREVET EUROPEEN

(11) **EP 2 831 081 B1**
(45) Date de publication et mention de la délivrance du brevet: **19.10.2016**
(21) Numéro de dépôt: 13716977.7
(22) Date de dépôt: 26.03.2013
(51) Int. Cl.: C07D 487/22, C09K 11/59, A61K 41/00, A61K 31/409, B82Y 5/00, A61P 35/00

(54) **NANOPARTICULES DE SILICIUM POREUX FONCTIONNALISEES ET LEURS UTILISATIONS EN THERAPIE PHOTODYNAMIQUE**
FUNKTIONALISIERTE PORÖSE SILICIUMNANOPARTIKEL UND VERWENDUNG DAVON IN DER PHOTODYNAMISCHEN THERAPIE
FUNCTIONALIZED POROUS SILICON NANOPARTICLES AND USE THEREOF IN PHOTODYNAMIC THERAPY

(30) Priorité: 27.03.2012 FR 1252703
(43) Date de publication de la demande: 04.02.2015
(73) Titulaire: Centre National de la Recherche Scientifique (CNRS), 75794 Paris Cedex 16 (FR); Université Montpellier 2, Sciences et Techniques, 34095 Montpellier Cedex 5 (FR); The Regents of the University of California, Oakland, CA 94607 (US); Ecole Nationale Supérieure de Chimie de Montpellier, 34296 Montpellier (FR)
(72) Inventeur: CUNIN, Frédérique, F-34000 Montpellier (FR); DURAND, Jean-Olivier, F-34250 Palavas-les-Flots (FR); SAILOR, Michael J., La Jolla, CA 92093-0358 (US); GARCIA, Marcel, F-34730 Prades-le-Lez (FR); SECRET, Emilie, F-27200 VERNON (FR); GARY-BOBO, Magali, F-34170 Castelnau-le-Lez (FR); MAYNADIER, Marie, F-34800 Ceyras (FR); MORERE, Alain, F-34980 Saint Gely du Fesc (FR)
(74) Mandataire: Hirsch & Associés
(86) Numéro de dépôt international: PCT/EP2013/056423
(87) Numéro de publication internationale: WO 2013/144154

(56) Documents cités:
- FR-A1- 2 935 974
- OUAHIBA HOCINE ET AL: "Silicalites and Mesoporous Silica Nanoparticles for photodynamic therapy", INTERNATIONAL JOURNAL OF PHARMACEUTICS, vol. 402, no. 1-2, 15 décembre 2010 (2010-12-15), pages 221-230, XP055039772, ISSN: 0378-5173, DOI: 10.1016/j.ijpharm.2010.10.004
- BREVET D ET AL: "Mannose-targeted mesoporous silica nanoparticles for photodynamic therapy", CHEMICAL COMMUNICATIONS - CHEMCOM; [6015D], ROYAL SOCIETY OF CHEMISTRY, GB, no. 12, 18 février 2009 (2009-02-18), pages 1475-1477, XP002607737, ISSN: 1359-7345, DOI: 10.1039/B900427K cité dans la demande
- EMILY K. LUMLEY ET AL: "Comparison of Photo-oxidation Reactions in Batch and a New Photosensitizer-Immobilized Microfluidic Device", ORGANIC LETTERS, vol. 14, no. 22, 16 novembre 2012 (2012-11-16), pages 5724-5727, XP55062710, ISSN: 1523-7060, DOI: 10.1021/ol3023424

## Description

La présente invention concerne des nanovecteurs permettant simultanément de réaliser le ciblage, l'imagerie et le traitement par thérapie photodynamique de cellules cancéreuses. En particulier elle concerne de nouvelles nanoparticules biodégradables à base de silicium contenant une variété de molécules photosensibilisantes, qui ont la capacité de cibler les cellules pathologiques et d'induire la mort cellulaire sous l'action d'une excitation dans le proche infra-rouge (> 600 nm) en mono et biphotonique. L'invention concerne également un procédé de traitement de certaines pathologies, et notamment des cancers, comprenant l'administration de nanoparticules biodégradables à base de silicium contenant au moins une molécule photosensibilisante et au moins une molécule de ciblage, cette administration étant suivie d'un traitement par une irradiation dans l'infra-rouge.

La thérapie photodynamique se fonde sur l'utilisation de certaines molécules thérapeutiques appelées photosensibilisateurs qui, lorsqu'elles sont activées à l'aide d'une source lumineuse de longueur d'onde appropriée, dans le domaine du visible ou du proche infra-rouge, transmettent leur énergie en excès à l'oxygène moléculaire les environnant. Ces photosensibilisateurs sont habituellement associés à des molécules de ciblage de telle sorte qu'elles vont préférentiellement se localiser dans les tissus malins. L'agent thérapeutique photosensensibilisant, lorsqu'il est activé par la lumière, convertit l'oxygène moléculaire à l'état fondamental triplet (³O₂) en oxygène singulet (¹O₂) hautement cytotoxique, et en autres espèces oxydantes réactives (O₂•-, OH•). Ces espèces oxygénées réactives, et plus particulièrement l'oxygène singulet, sont toxiques pour les cellules qui les entourent et entraînent la destruction des tissus cancéreux dans leur proche environnement : elles oxydent les membranes cellulaires et entraînent ainsi des lésions irréversibles des cellules contenant le photosensibilisateur. C'est un traitement prometteur utilisé en clinique depuis une dizaine d'années. Les avantages de la thérapie photodynamique sont la possibilité de répéter les traitements sans toxicité cumulée (comparée à la radiothérapie), son faible coût, sa simplicité de mise en oeuvre, et son applicabilité à un grand nombre de maladies (cardiovasculaires, dermatologiques et ophtalmiques). Puisque le traitement nécessite de la lumière pour être effectif, il est localisé et présente peu de toxicité systémique. Pour les tumeurs malignes, la thérapie photodynamique peut être plus efficace et moins pénible que d'autres thérapies.

La thérapie photodynamique se fonde sur une double sélectivité : en premier lieu l'irradiation sélective des tissus concernés et en second lieu la sélectivité relative du photosensibilisateur pour les tissus cibles. En l'absence d'irradiation, les photosensibilisateurs étant peu toxiques pour les cellules, leur diffusion dans l'organisme n'entraîne que peu d'inconvénients.

Un photosensibilisateur, pour pouvoir être employé in vivo doit posséder plusieurs qualités, et notamment, il doit pouvoir être facilement vectorisé vers les tissus cancéreux, il doit être hydrosoluble, facile à produire, non toxique en l'absence d'irradiation, stable vis-à-vis des enzymes circulantes, présentant un bon tropisme pour les cellules tumorales et il doit être éliminé rapidement des tissus sains. Une autre contrainte de ces formulations est de pouvoir conserver l'efficacité des photosensibilisateurs, c'est-à-dire leur capacité à transformer l'oxygène moléculaire qui les entoure en espèces oxygénées réactives. En effet, certaines formulations interagissent avec les états excités du photosensibilisateur et réduisent son efficacité.

### Etat de la technique antérieure

Les traitements actuels de maladies et cancers par thérapie utilisant la génération d'espèces oxydantes (ROS et oxygène singulet), tels que la thérapie photodynamique procèdent par photoexcitation de molécules organiques photosensibilisantes de type porphyrines. L'utilisation des ces molécules organiques photosensibilisantes est toutefois soumise aux limites suivantes: les molécules organiques photosensibilisantes sont hydrophobes et nécessitent un système de délivrance pour une application clinique, notamment des formulations particulières, en particulier sous forme de suspensions colloïdales, de liposomes, de nanoparticules. Ces molécules ne ciblent pas efficacement les cellules tumorales, elles présentent un temps de résidence important dans les tissus "normaux" et peuvent être activées par la lumière visible, ce qui engendre des effets secondaires de destruction des cellules saines des tissus superficiels (peau, oeil) par exposition à la lumière ambiante et une photo-sensibilité prolongée des patients.

Le système photosensilisateur idéal doit présenter 1/ un rendement quantique en oxygène singulet et ROS élevé, 2/ une conversion optique importante aux grandes longueurs d'onde (700-1200 nm), 3/ une toxicité minimale dans l'obscurité et une toxicité à la lumière visible et UV minimale dans le temps, 4/ une localisation préférentielle dans les cellules malades.

Une approche prometteuse développée par bon nombre de chercheurs pour surmonter les difficultés énumérées ci-dessus consiste à incorporer les molécules photosensibilisantes (porphyrines et autres) dans des nanoparticules de céramique, de silices, d'oxydes ou de polymères. Les nanoparticules de ces matériaux utilisées comme système hôte permettent en particulier d'améliorer la biodisponibilité des molécules photosensibilisantes hydrophobes, et le ciblage par des ligands spécifiques dont les récepteurs sont sur-exprimés dans les cellules malades.

Plusieurs documents décrivent des nanostructures de silice mésoporeuses pour des applications thérapeutiques et de ciblage. Notamment, WO 2011073054 et FR 2935974 décrivent des nanoparticules de silice poreuses obtenues par voie sol-gel pour des applications thérapeutiques et en thérapie photodynamique, mais n'ayant pas les propriétés physiques des nanoparticules de silicium poreux de l'invention. Les porphyrines utilisées dans FR 2935974 sont similaires aux porphyrines utilisées dans la présente invention, mais dans le dispositif FR 2935974 les nanostructures auxquelles ces porphyrines sont incorporées ne peuvent pas être excitées dans le proche infra-rouge (en mono et bi-photonique).

La silice est une forme de dioxyde de silicium SiO2 que l'on trouve dans la nature, tandis que le silicium poreux est préparé à partir de silicium cristallin, matériau semiconducteur. Lorsque que le silicium est rendu mésoporeux, et donc structuré à l'échelle nanométrique, il possède des propriétés physiques, en particulier optiques uniques (photoluminescence, etc...) dûe à des effets de confinement quantique. Ces propriétés électroniques et physiques n'existent pas pour une silice poreuse, qui est un matériau isolant électronique et amorphe.

Plusieurs documents décrivent des micro et nanostructures à base de silicium poreux pour des applications thérapeutiques et de ciblage. Notamment WO 2010096733, WO 2009009563 et WO 2006050221 décrivent des micro et nanostructures de silicium poreux pour des applications thérapeutiques, vectorisation de molécules et imagerie, mais ne décrivent pas de transfert d'énergie du silicium poreux à des molécules photosensibilisantes, ni d'application en thérapie photodynamique. Ji-Ho Park et al., Nature Materials, 8, April 2009, 331-336 décrit l'utilisation de nanoparticules de silicium poreux pour la visualisation par luminescence de tissus tumoraux. Les nanoparticules de silicium poreux luminescent peuvent être excitées dans l'infra-rouge et notamment par excitation biphotonique.

Un photosensibilisant à base de Qdot CdSe et de porphyrine a été décrit dans J. Mater. Chem., 2011, 21, 2455-2458. Ce matériau peut être utilisé en irradiation biphotonique pour des applications en photothérapie dynamique. Toutefois, l'utilisation de cadmium pose des problèmes de toxicité.

La communication Euromat, 2011, Montpellier, France, E.Secret et al., « Porous silicon nanoparticles functionalized for photodynamic therapy » décrit le greffage du mannose sur les nanoparticules de silicium poreux, et le greffage d'une porphyrine sur les nanoparticules de silicium poreux hydrosilylé fonctionnalisé par l'acide undécènoïque. Toutefois, la méthode de greffage employée n'est pas satisfaisante dans la mesure où il a été constaté que le greffage de la porphyrine n'était pas suffisamment stable et ne permettait pas l'acheminement satisfaisant du photosensibilisateur jusqu'aux cellules cibles. En outre, il n'est ni mentionné ni suggéré dans ce document de transfert d'énergie du silicium poreux à des molécules photosensibilisantes et/ou la possibilité d'une excitation biphotonique.

C'est donc avec étonnement que les inventeurs ont découvert que des nanoparticules de silicium poreux comportant au moins une molécule photosensibilisante greffée de façon covalente, telle qu'une porphyrine par exemple, étaient susceptibles d'être excitées dans le proche infra-rouge, en mode mono et bi-photonique, et pouvaient donc être utilisées dans des traitements de thérapie photodynamique. En particulier, les inventeurs suggèrent que dans de telles nanoparticules, lorsqu'elles sont soumises à une excitation dans le proche infra-rouge, un transfert d'énergie est opéré du silicium poreux vers les molécules photosensibilisantes. En outre, ces nanoparticules peuvent également être greffées par des molécules de ciblage qui permettent une spécificité d'action à l'encontre des cellules cancéreuses.

### Résumé de l'invention

Un premier objet de l'invention consiste en des nanoparticules répondant à la formule (I) : 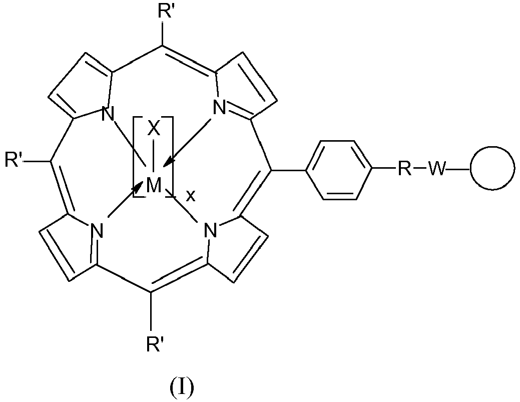 dans laquelle : 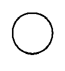
représente une nanoparticule de silicium poreux,
x représente un entier choisi parmi : 0 et 1,
M représente un atome de métal choisi parmi les métaux de transition,
X représente un groupement choisi parmi : un halogénure, un anion d'un acide carboxylique pharmaceutiquement acceptable,
R représente un groupement choisi parmi : une urée (-NH-CO-NH-), une thiourée (-NH-CS-NH-),
W représente un groupement alcane di-yle en C1-C12,
R' représente un groupement choisi parmi :

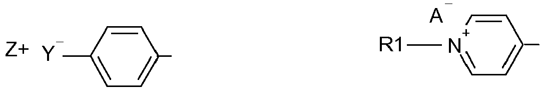

Z⁺ représente un cation organique ou minéral pharmaceutiquement acceptable,
Y⁻ représente un groupement qui peut être choisi parmi : -COO⁻, -SO₃⁻
A⁻ représente un anion qui peut être choisi parmi : un halogénure, un anion d'un acide carboxylique pharmaceutiquement acceptable,
R1 représente un alkyle en C1 à C10.

Selon un mode de réalisation préféré les nanoparticules répondent à la formule (Ia) :

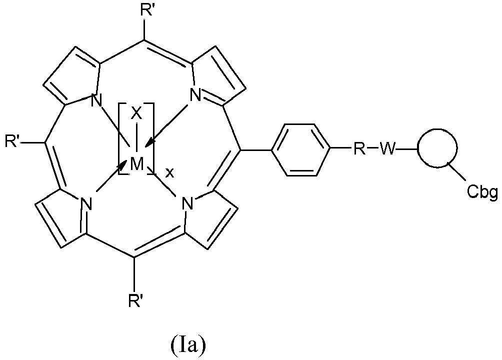

Dans laquelle Cbg représente une molécule de ciblage spécifique des tissus néoplasiques.

Selon un mode de réalisation préféré les nanoparticules ont une taille allant de 20 à 200 nm, avantageusement de 50 à 190 nm, encore mieux, de 100 à 175 nm, et de préférence de 140 à 160 nm et la taille des pores va de 5 à 50 nm, avantageusement de 10 à 30 nm.

Selon un mode de réalisation préféré dans les formules (I) et (Ia) x représente 0.

Selon un mode de réalisation préféré dans les formules (I) et (Ia) l'une ou plusieurs des caractéristiques suivantes sont vérifiées :
X représente un groupement choisi parmi : Cl⁻, Br⁻, I⁻, acétate, propionate, butyrate, ascorbate, benzoate, cinnamate, citrate, fumarate, glycolate, malonate, tartrate, malate, maléate, mandélate, tosylate,
W représente un groupement -(CH₂)₃-.
R1 représente un groupement choisi parmi : un alkyle en C1-C3,
Z⁺ représente un cation qui peut être choisi parmi : K⁺, Li⁺, Na⁺, NH₄⁺,
A représente un anion qui peut être choisi parmi : Cl⁻, Br⁻, I⁻, acétate, propionate, butyrate, ascorbate, benzoate, cinnamate, citrate, fumarate, glycolate, malonate, tartrate, malate, maléate, mandélate, tosylate,
R' est choisi parmi : 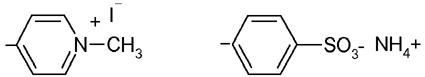
Cbg, lorsqu'il est présent, est choisi parmi les sucres et les dérivés de sucres. Les nanoparticules préférées sont choisies parmi :

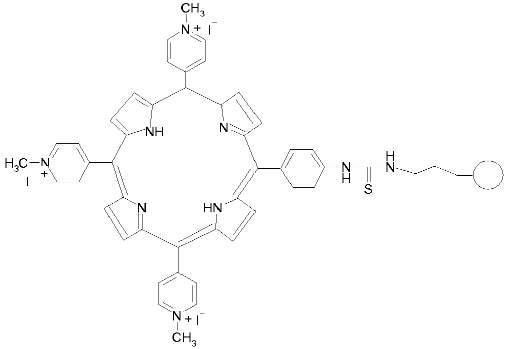

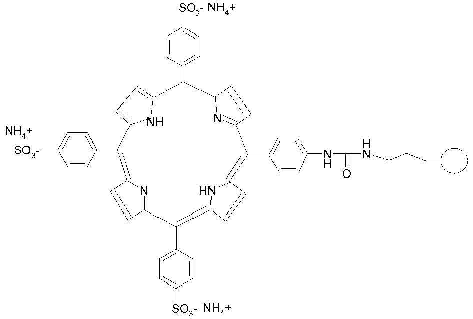

L'invention concerne encore un procédé de fabrication de nanoparticules comprenant les étapes de :
(i) fourniture de nanoparticules de silicium poreux,
(ii) fonctionnalisation des nanoparticules de silicium poreux par des groupements comportant au moins une fonction NH₂ ou au moins une fonction isocyanate, isothiocyanate, ou semicarbazide
(iii) fourniture et greffage d'une molécule photosensibilisante, porteuse d'un groupement complémentaire de celui porté par la nanoparticule, de façon à former une liaison urée ou thiourée, et éventuellement
(iv) greffage par au moins une molécule de ciblage.

Selon une variante préférée, le procédé comporte les étapes de :
(i)
   a- gravure électrochimique de plaquettes de silicium monocristallin dans une solution éthanolique d'acide fluorhydrique (HF),
   b- élimination du film poreux et traitement par ultrasons,
(ii)
   a- oxydation ménagée suivie d'une silanisation de façon à produire des groupements Si-OH et des espèces SiO₂ à la surface des nanoparticules de silicium poreux, et/ou
   b- hydrosilylation,
(iii) greffage des nanoparticules par une molécule photosensibilisante de type porphyrine répondant à la formule (II) dans laquelle Q représente un groupement choisi parmi : -NH₂, -N=C=O, -N=C=S, 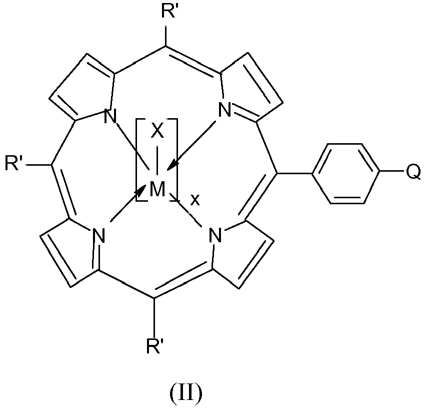
(iv) greffage des nanoparticules par un sucre ou un dérivé de sucre à l'aide d'un groupement phénylsquarate ou d'un groupement semicarbazone.

L'invention concerne encore des nanoparticules décrites ci-dessus, pour leur utilisation pour la détection, le traitement, le suivi, la prévention, pour retarder l'apparition et/ou la récidive d'une pathologie choisie parmi les cancers, les tumeurs, les maladies prolifératives cellulaires, les maladies de la peau.

Elle concerne également une composition de médicament comprenant des nanoparticules telles que décrites ci-dessus dans un support pharmaceutiquement acceptable.

Elle concerne également une composition cosmétique comprenant des nanoparticules telles que décrites ci-dessus dans un support pharmaceutiquement acceptable.

Elle concerne encore un kit pour la détection, le traitement, le suivi, la prévention, pour retarder l'apparition et/ou la récidive d'une pathologie choisie parmi les cancers, les tumeurs, les maladies prolifératives cellulaires comprenant :
- des nanoparticules de silicium poreux greffées par l'intermédiaire d'une liaison covalente par au moins une molécule photosensibilisante, ou une composition de médicament les comprenant
   et
- des moyens permettant une irradiation biphotonique dans l'infra-rouge.

En particulier l'invention concerne un kit pour la détection, le traitement, le suivi, la prévention, pour retarder l'apparition et/ou la récidive d'une pathologie choisie parmi les cancers, les tumeurs, les maladies prolifératives cellulaires comprenant :
- des nanoparticules telles que définies ci-dessus, ou une composition telle que définie ci-dessus,
   et
- des moyens permettant une irradiation dans l'infra-rouge.

L'invention concerne des dispositifs et agents thérapeutiques pour le traitement non invasif d'un grand nombre de maladies et de cancers.

L'invention concerne en particulier des nanostructures ou nanoparticules de silicium poreux biodégradables et non toxiques portant des molécules organiques photosensibilisantes. Les nanostructures de silicium poreux permettent aux molécules photosensensibilisantes de produire des espèces oxydantes, dont l'oxygène singulet, fortement cytotoxiques, lorsqu'elles sont irradiées par de la lumière proche infra-rouge (en mode mono et/ou biphotonique) pour le traitement non invasif de maladies diverses incluant le cancer. La présence des nanostructures hôtes de silicium poreux, permet aux molécules photosensibilisantes de générer des espèces oxydantes par photo-excitation dans le proche infra-rouge. Lorsqu'elles sont isolées ou encapsulées dans d'autres types de matériaux hôtes, tels que les matériaux auxquels elles sont associées dans l'art antérieur, ces molécules photosensibilisantes ne sont pas excitables dans le proche infra-rouge à des puissances acceptables pour un organisme vivant. On peut les exciter en biphotonique mais à des puissances très élevées ce qui produit des photo-dommages au niveau des tissus. L'utilisation de la lumière proche infra-rouge a l'avantage de permettre une pénétration dans les tissus vivants plus importante que d'autres longueurs d'ondes de lumière, pour un traitement thérapeutique non invasif. Les nanostructures de silicium poreux de taille et de texture contrôlées sont préparées par voie électrochimique, sonication et filtration. Les molécules photosensibilisantes sont liées aux nanostructures hôtes de manière covalente par des chimies de greffage nouvelles sur ce type de matériau hôte. Des agents de ciblage des cellules malades peuvent être également greffés ou pas sur les nanostructures de silicium poreux par des méthodes originales. Ces nanostructures multifonctionnelles sont internalisées par les cellules cancéreuses in vitro. Les espèces oxydantes et l'oxygène singulet générés par excitation dans le proche infra-rouge (750 nm, biphotonique) détruisent de manière irréversible les cellules malades.

L'invention permet l'utilisation de la lumière proche infra-rouge (> 600 nm) pour l'excitation de nanoparticules de silicium poreux contenant des molécules photosensibilisantes génératrices d'espèces oxydantes (dont l'oxygène singulet) dans le but d'induire la mort cellulaire. Grâce à ses propriétés de confinement quantique, le silicium poreux peut subir des réactions de transfert d'énergie. Ces processus de transfert d'énergie peuvent être très efficaces en raison du temps de vie élevé (microsecondes) des excitons et de sa surface spécifique importante. Le silicium poreux peut aussi générer lui-même des espèces oxydantes (ROS et oxygène singulet).

L'intérêt d'une excitation dans le proche infra-rouge est la pénétration plus importante des tissus par la lumière, ce qui permet le traitement de diverses maladies et du cancer dans des tissus plus profonds, avec la possibilité d'une thérapie non invasive. L'excitation dans le proche infra-rouge permet aussi de limiter les effets secondaires dus à la lumière dans les tissus traités par thérapie photodynamique.

L'invention permet l'encapsulation et la vectorisation de la molécule photosensibilisante. Les molécules photosensibilisantes sont liées de manière covalente aux nanoparticules de silicium poreux. Cette liaison a été rendue possible par la mise au point d'une méthode de greffage covalent appropriée. Les nanoparticules de silicium poreux sont également modifiées par des molécules de ciblage qui leur permettent de reconnaitre sélectivement des cellules cibles in-vivo.

Le greffage covalent des molécules photosensibilisantes par des chimies originales décrites dans l'invention permet le contrôle de la vectorisation de ces molécules photosensibilisantes jusqu'aux cellules cibles grâce au ciblage par des ligands spécifiques dont les récepteurs sont sur-exprimés dans les cellules malades et il permet également d'améliorer la biodisponibilité des molécules hydrophobes par leur encapsulation

Les nanoparticules de silicium poreux offrent une grande surface spécifique avec la capacité de lier et d'être chargées avec plusieurs molécules ou complexes, identiques ou différents qui sont délivrés dans les tissus in vivo.

En outre, leurs propriétés luminescentes permettent un suivi de leur présence dans l'organisme et une utilisation dans le diagnostic.

Lorsqu'elles sont présentes en quantité compatible avec des applications thérapeutiques, les nanoparticules de silicium poreux peuvent circuler dans le sang plusieurs jours sans évidence de toxicité. Les particules de silicium poreux non fonctionalisées seules n'induisent pas de réponse toxique rédhibitoire pour envisager leur utilisation en clinique.

Les nanoparticules de silicium poreux sont biocompatibles et biodégradables et se dégradent en composés éliminés par clairance rénale avec une absence de toxicité. Le silicium poreux est une alternative très attractive aux autres semi-conducteurs nanostructurés de types Qdots, à base de métaux lourds, ayant des propriétés de confinement quantique intéressantes, mais qui sont très toxiques. Le silicium à l'état de trace est un élément nécessaire à la croissance des os et au collagène. Les produits ultimes de dégradation du silicium poreux sont des oxo anions d'acide orthosilicique, Si(OH)₄. Ils constituent les formes biodisponibles du silicium. L'acide orthosilicique est naturellement présent dans de nombreux tissus et un excès d'acide silicique sera éliminé efficacement du corps par les reins. Le silicium poreux possède des propriétés très avantageuses pour des applications in vivo: sa faible toxicité, sa surface spécifique, ses pores et son volume de pores de taille modulable, sa compatibilité avec la croissance de cellules. Les nanoparticules de silicium poreux répondent au cahier des charges du système photosensilisateur idéal (encapsulation, vectorisation, excitation dans le proche infra-rouge, sensibilité limitée à la lumière).

L'invention permet la biodégradation et l'élimination avec une cinétique contrôlée des particules hôtes vecteurs du silicium poreux.

Les synthèses nécessaires à la fabrication de l'invention sont courtes dans le temps et faciles à mettre en oeuvre.

### Description détaillée

### - Les nanoparticules :

L'invention concerne des nanoparticules répondant à la formule (I) ci-dessous 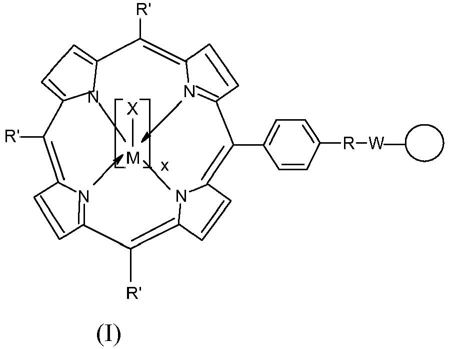 dans laquelle : 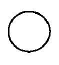
représente une nanoparticule de silicium poreux,
x représente un entier choisi parmi : 0 et 1,
M représente un atome de métal choisi parmi les métaux de transition,
X représente un groupement choisi parmi : un halogénure, un anion d'un acide carboxylique pharmaceutiquement acceptable,
R représente un groupement choisi parmi : une urée (-NH-CO-NH-), une thiourée (-NH-CS-NH-),
W représente un groupement alcane di-yle en C1-C12,
R' représente un groupement choisi parmi :

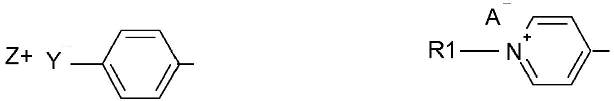

Z⁺ représente un cation organique ou minéral pharmaceutiquement acceptable,
Y⁻ représente un groupement qui peut être choisi parmi : -COO⁻, -SO₃⁻
A⁻ représente un anion qui peut être choisi parmi : un halogénure, un anion d'un acide carboxylique pharmaceutiquement acceptable,
R1 représente un alkyle en C1 à C10.

Sur la formule (I), seul le greffage par une porphyrine est représenté, toutefois la définition de cette formule inclut la possibilité d'un greffage par d'autres groupements tels que des molécules de ciblage par exemple.

Par nanoparticules de silicium poreux on entend des structures poreuses de taille allant de 1 nm à 900 nm, à base de silicium. Les nanoparticules de silicium poreux sont connues ainsi que des procédés permettant leur fabrication. Les documents suivants décrivent des nanoparticules de silicium poreux utilisables dans la présente invention ainsi qu'un procédé pour leur préparation : WO 2010096733, WO 2009009563 et WO 2006050221, Ji-Ho Park et al., Nature Materials, 8, April 2009, 331-336. De façon préférentielle, la taille des nanoparticules de silicium poreux est de 20 à 200 nm, avantageusement de 50 à 190 nm, encore mieux, de 100 à 175 nm, et de préférence de 140 à 160 nm. La taille des nanoparticules est mesurée par diffusion dynamique de la lumière (DLS), microscopie électronique à balayage (MEB) et microscopie électronique à transmission (TEM). La taille des pores est préférentiellement d'une valeur allant de 5 nm à 50 nm, avantageusement de 10 nm à 30 nm. La taille des pores peut être mesurée par microscopie électronique à balayage (MEB) et analyse d'adsorption/désorption d'azote.

Un groupement alkyle en C1-C10 est une chaîne hydrogénocarbonée, linéaire, ramifiée ou cyclique comportant de 1 à 10 atomes de carbone.

Lorsque x représente 0, le composé de formule (I) est un dérivé de porphyrine et les groupements (M-X) sont remplacés par deux atomes d'hydrogène. Lorsque x représente 1, le composé de formule (I) est un dérivé de métalloporphyrine.

Selon une variante de l'invention représentée par la formule (Ia), la nanoparticule de formule (I) comporte en outre au moins une molécule de ciblage Cbg.

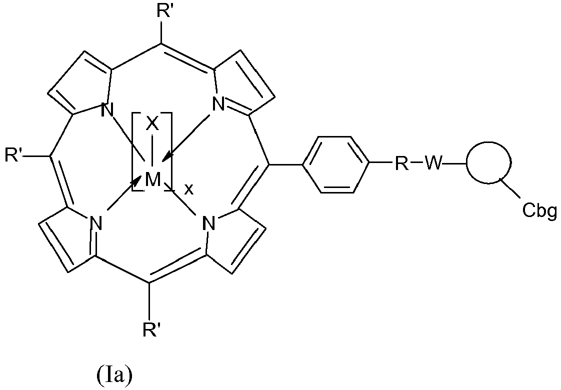

Cbg est préférentiellement choisie parmi les molécules de ciblage spécifiques des tissus néoplasiques, c'est-à-dire des biomolécules dont les récepteurs sont surexprimés par les cellules cancéreuses, ou à la surface des cellules cancéreuses. D'une façon générale, les molécules de ciblage Cbg peuvent être choisies parmi : l'acide folique, les peptides, les hydrates de carbone, les anticorps.

Pour simplifier la représentation, seulement une porphyrine et une molécule de ciblage sont représentées sur les formules (I) et (Ia). Toutefois, l'invention définie par les formules (I) et (Ia) inclut le greffage de plusieurs porphyrines, identiques ou différentes, et de plusieurs molécules de ciblage, identiques ou différentes, sur une nanoparticule de silicium.

Dans les formules (I) et (Ia), les variables définies ci-dessus sont avantageusement choisies, de façon indépendante, suivant les règles suivantes :
Selon une première variante préférée x représente 0.

Lorsque x représente 1, de préférence, M représente un atome de métal choisi parmi : Zn, Pt, Pd, Mn, Gd, Ni, Cr, Ru. De préférence M est choisi parmi Pd et Zn.

De préférence, X représente un groupement choisi parmi : Cl⁻, Br⁻, I⁻, acétate, propionate, butyrate, ascorbate, benzoate, cinnamate, citrate, fumarate, glycolate, malonate, tartrate, malate, maléate, mandélate, tosylate, et encore plus préférentiellement : Cl⁻, Br⁻, I⁻, acétate, tosylate.

De préférence W représente un groupement -(CH₂)₃-.

Avantageusement R1 représente un groupement choisi parmi : un alkyle en C1-C3, comme par exemple un méthyle, un éthyle, un n-propyle, un isopropyle, et encore plus avantageusement un méthyle.

De préférence, Z⁺ représente un cation qui peut être choisi parmi : K⁺, Li⁺, Na⁺, NH₄⁺.

De préférence A⁻ représente un anion qui peut être choisi parmi : Cl⁻, Br⁻, I⁻, acétate, propionate, butyrate, ascorbate, benzoate, cinnamate, citrate, fumarate, glycolate, malonate, tartrate, malate, maléate, mandélate, tosylate, et encore plus préférentiellement. Cl⁻, Br⁻, F⁻, acétate, tosylate.

De préférence R' est choisi parmi :

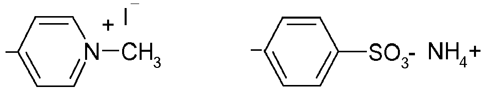

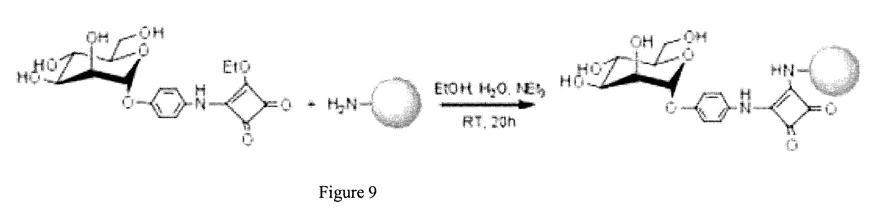

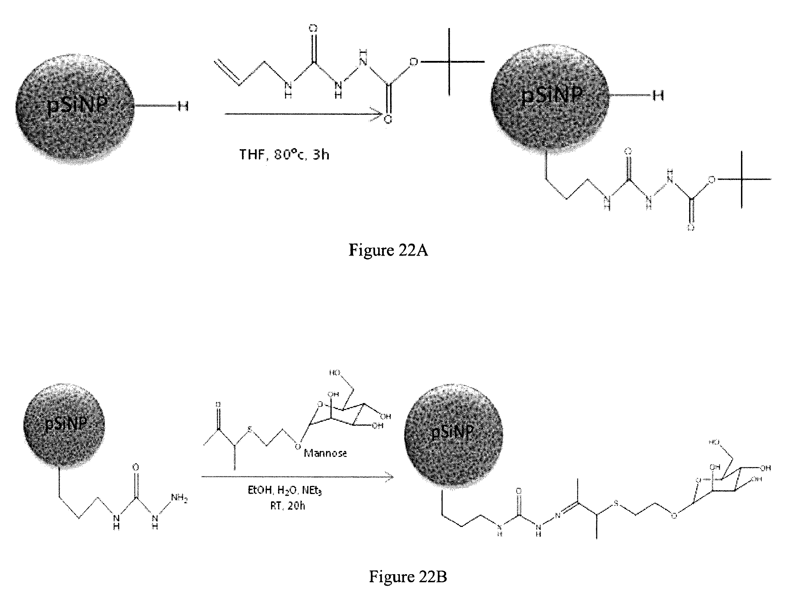

Parmi les molécules de ciblage Cbg qui peuvent être greffées sur les nanoparticules de l'invention on peut mentionner :
- des sucres et des dérivés de sucre comme le mannose, qui peut être greffé sur des nanoparticules de silicium poreux à l'aide d'un linker polyéthylèneimine ou polyéthylène glycol PEG. D'autres voies de greffage consistent à utiliser un dérivé phénylsquarate de sucre, tel que le phénylsquarate-[alpha]-mannose comme illustré sur la figure 9 et dans la partie expérimentale, ou bien un mannose-fonctionnalisé cétone comme illustré sur la figure 22B et dans la partie expérimentale.
- des peptides tels que des peptides CREKA iRGD ou des peptides à cible hormonale (LH-RH).
- des glycodendrimères portant plusieurs dérivés monosaccharides ou disaccharides tels que le mannose, le mannose-6-phosphate, le glucose, le galactose,...

Les nanoparticules de formule (I) préférées appartiennent à la liste suivante :

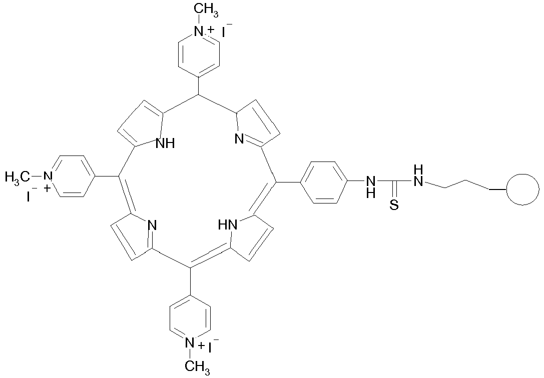

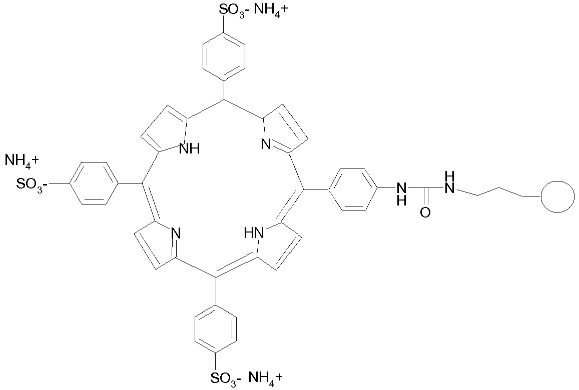

### - La fabrication des nanoparticules de l'invention:

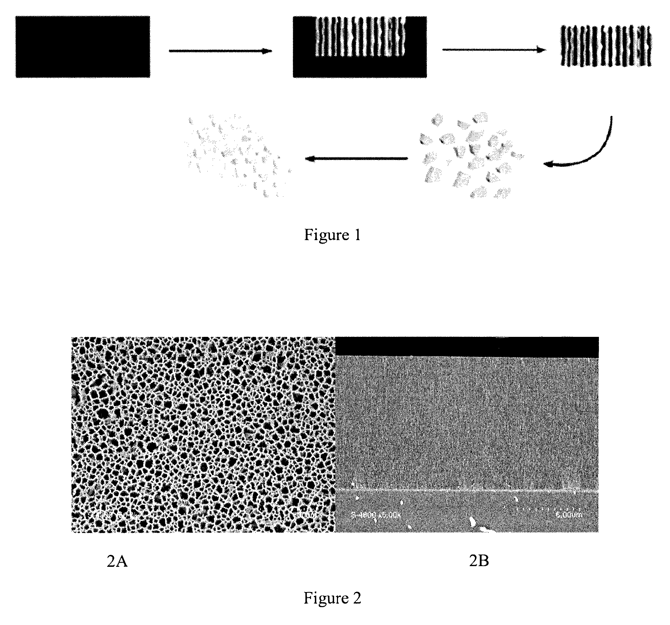

Les nanoparticules de l'invention peuvent notamment être fabriquées par un procédé illustré sur les figures 1, 6, 7, 8, 9, 14A 20, 22A et 22B.

Dans une première étape on prépare des nanoparticules de silicium poreux, suivant des méthodes connues de l'homme du métier.

Ces nanoparticules sont ensuite fonctionnalisées pour porter à leur surface au moins un groupement NH₂ ou au moins un groupement isocyanate, isothiocyanate ou semicarbazide .

Les nanoparticules sont ensuite greffées par au moins une molécule photosensibilisante, notamment une porphyrine, en utilisant une molécule porteuse d'un groupement complémentaire de celui porté par la nanoparticule, de façon à former une liaison urée ou thiourée.

Contrairement à d'autres liaisons covalentes dont la synthèse a été testée sur ce type de structure, les liaisons urée et thiourée se forment dans des conditions de synthèse simples et préférentiellement à d'autres types de liaison entre la nanoparticule et la molécule photosensibilisante, telles que la physisorption par exemple.

Les nanoparticules sont ensuite avantageusement greffées par au moins une molécule de ciblage.

### □ Préparation des nanoparticules de silicium poreux :

Cette étape fait appel à des méthodes bien connues de l'homme du métier. De telles méthodes ont notamment été décrites dans WO 2010096733, WO 2009009563 et WO 2006050221, Ji-Ho Park et al., Nature Materials, 8, April 2009, 331-336.

Les nanoparticules de l'invention sont à base de silicium

Le silicium est l'élément chimique de symbole Si et de numéro atomique 14. Le silicium se trouve occasionnellement en tant qu'élément pur libre dans la nature, mais il est plus largement présent sous diverses formes de dioxyde de silicium (silice) ou de silicates. Le silicium est utilisé dans l'industrie électronique sous forme de silicium de haute pureté pour la formation de plaques. Le silicium pur est utilisé pour produire des plaques de silicium ultra-pur utilisées dans l'industrie des semi-conducteurs, dans l'électronique et dans des applications photovoltaïques. Le silicium ultra-pur peut être dopé avec d'autres éléments pour ajuster sa réponse électrique en contrôlant le nombre et la charge (positive ou négative) des porteurs de courant. En photonique, le silicium peut être utilisé comme un support pour laser Raman à onde continue pour produire une lumière cohérente. De préférence, le silicium utilisé dans la mise en oeuvre de l'invention est dopé. Avantageusement, le silicium utilisé dans la mise en oeuvre de l'invention comporte un dopage positif au bore. Par exemple un dopage au bore d'environ 1 atome sur 1000 peut être employé.

L'oxyde de silicium consiste généralement en un atome de silicium lié à une seule espèce réactive de l'oxygène (par exemple, un radical). Ces composés d'oxyde de silicium sont utiles pour l'addition de carbone ou d'autres éléments chimiques dans lequel une liaison est formée entre l'oxygène et l'élément réactif ou une chaîne latérale chimique.

Le dioxyde de silicium se réfère au composé SiO₂ (également appelé silice). Le dioxyde de silicium est formé lorsque le silicium est exposé à l'oxygène (ou de l'air). Une couche mince (environ 1 nm ou 10 A) d'oxyde natif est formée sur la surface lorsque le silicium est exposé à l'air dans les conditions ambiantes. Des températures plus élevées et des environnements alternatifs sont utilisés pour produire des couches de dioxyde de silicium sur du silicium. Le dioxyde de silicium est inerte et inoffensif.

Les nanoparticules de silicium poreux utilisées dans la présente invention sont avantageusement préparées par gravure électrochimique de plaques de silicium monocristallin dans une solution éthanolique d'acide fluorhydrique (HF) suivie du détachement du film poreux et le traitement par ultrasons selon une procédure illustrée sur la figure 1 et connue de l'homme du métier (Sailor, M. J. et al., Adv. Funct. Mater. 2009, 19 (20), 3195-3208 ; Bley, R. A. et al., Chem. Mat. 1996, 8 (8), 1881-1888).

La taille d'une nanoparticule joue un rôle crucial dans sa biocompatibilité. Par exemple, les particules dans la gamme 20-200 nm ont tendance à présenter les plus longues durées de circulation dans le sang (Park, J.-H. et al., Nature Mater. 2009, 8, 331-336 ; Ruoslahti, E. et al., J. Cell Biol. 2010, 188 (6), 759-768 ; Lundqvist, M. et al., Nanoparticle size and surface properties determine the protein corona with possible implications for biological impacts. Proceedings of the National Academy of Sciences 2008, 105 (38), 14265 ; De Jong, W. H. et al., Biomaterials 2008, 29 (12), 1912-1919 ; Akerman, M. E. et al., Proc Natl Acad Sci U S A 2002, 99 (20), 12617-21 ; Decuzzi, P. et al., J. Control. Release 2010, 141 (3), 320-327 ; Park, J.-H.et al. Small 2009, 5 (6), 694-700). Les nanoparticules présentent une cinétique d'internalisation cellulaire dépendant de leur taille, (Win, K. Y. et al., Biomaterials 2005, 26 (15), 2713-2722) et une cytotoxicité dépendante de leur taille a été observée pour les concentrations de formulations de nanoparticules de masse équivalente (Choi, J. et al., J. App. Tox. 2009, 29 (1), 52-60)). En outre, le taux de dégradation d'une nanoparticule dépend souvent du diamètre des particules, ce qui affecte à son tour le profil de libération de médicament d'une nanoparticule thérapeutique.

Le Si poreux est un produit résultant du traitement électrochimique de simples plaquettes de Si cristallins dans une solution d'électrolyte d'acide fluorhydrique. La morphologie des pores et la taille des pores peuvent être modifiées en contrôlant la densité de courant, le type et la concentration de dopant, l'orientation cristalline de la plaquette de Si, et la concentration d'électrolyte. Le type de dopant dans la plaquette de silicium d'origine est important car elle détermine la disponibilité de la bande de valence des trous.

En général les caractéristiques du dopant peuvent être séparées en quatre groupes basés sur le type et la concentration du dopant: de type n, de type p, fortement dopée de type n, et fortement dopée de type p. Par fortement dopée, on entend les niveaux de dopant auxquels le comportement de conductivité du matériau est plus métallique que semiconducteur. L'application d'un courant anodique oxyde un atome de silicium de surface, qui est ensuite attaqué par le fluor. La formation de pores se produit lorsque des atomes de Si sont retirés sous la forme de SiF₄, qui réagit avec deux équivalents de F⁻ en solution pour former SiF₆²⁻. La porosité d'une couche de Si poreux est proportionnelle à la densité de courant appliquée, et elle est comprise typiquement entre 40 et 80%. Les pores se forment à l'interface Si/Si poreux, et une fois formées, la morphologie des pores ne change pas de manière significative pendant le reste du processus de gravure. Cependant, la porosité d'une couche en croissance peut être modifiée en changeant le courant appliqué. Le film va continuer à croître avec cette porosité nouvelle.

Cette propriété permet la construction de nanostructures en couches simplement en modulant le courant appliqué au cours d'une gravure électrochimique.

La capacité de contrôler facilement la taille des pores et des volumes poreux en ajustant les paramètres de courant lors de la gravure est une propriété unique de Si poreux qui est très utile pour les applications dans l'administration de médicaments.

### □ Fonctionnalisation de la surface des nanoparticules :

La chimie de surface joue un rôle important dans le greffage des nanoparticules et le contrôle des propriétés de dégradation de Si poreux in vivo. Après un traitement de gravure électrochimique, la surface des nanoparticules de Si est recouverte d'espèces réactives de type hydrure. Ces fonctionnalités chimiques fournissent un point de départ pour diverses réactions qui déterminent les taux de dissolution en milieu aqueux, permettent la fixation des molécules photosensibilisantes, des molécules de ciblage, et contrôlent les taux de libération de médicaments.

Les deux réactions de modification les plus importantes sont l'oxydation chimique et le greffage d'espèces Si-C.

Modification de la surface des nanoparticules de silicium poreux :
Les nanoparticules de silicium poreux sont tout d'abord chimiquement modifiées à l'aide de ligands fonctionnels.

Des molécules synthétiques de type porphyrines hydrophiles sont greffées de façon covalente aux nanoparticules de silicium poreux fonctionnalisées par ces ligands à l'aide de réactions chimiques originales. De façon avantageuse, des agents de ciblage sont également fixés aux nanoparticules de silicium poreux.

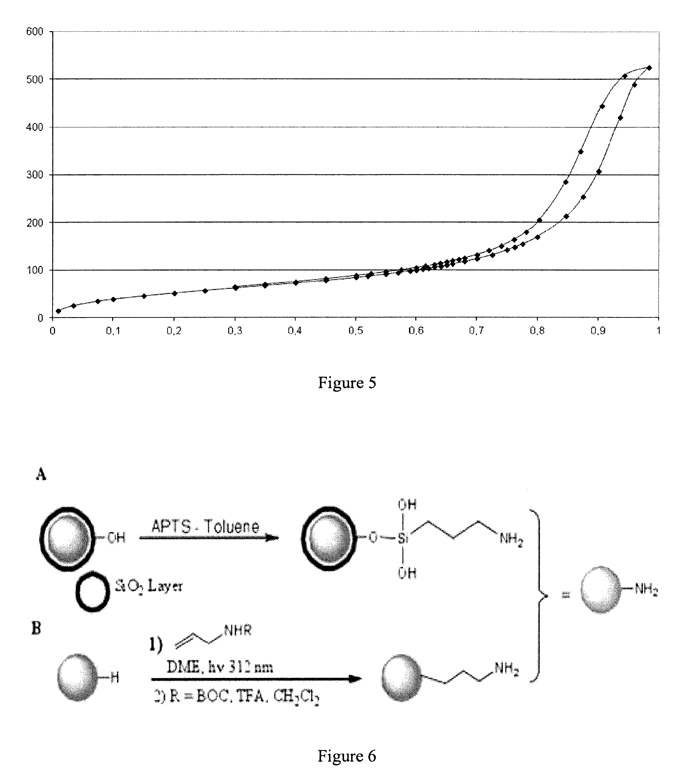

La modification chimique des nanoparticules de silicium poreux par des ligands fonctionnels:
Les nanoparticules fraîchement gravées de silicium poreux contiennent des liaisons Si-H sur leur surface et sont très hydrophobes. Deux modifications de surface principales ont été étudiées.
   - L'oxydation ménagée (oxydation thermique, oxydation par l'ozone, le sulfoxyde de diméthyle, ou le borate aqueux) suivie d'une silanisation : Des groupements Si-OH et des espèces SiO₂ sont produites à la surface des nanoparticules de silicium poreux. Ces groupements permettent de rendre les surfaces hydrophiles et biocompatibles. Comme illustré sur la figure 6A, une particule de silicium poreux traitée par oxydation présente une couche de SiO₂ en surface qui permet, par traitement par un agent silanisant tel que de l'aminopropyl triéthoxysilane, de greffer un groupement aminopropylsilanyl par une liaison covalente à la surface de la nanoparticule. Plus généralement, ce type de réaction permet la fixation de groupements aminoalkylsilanyl en surface des nanoparticules de silicium poreux avec un groupement W de taille variable.
- Hydrosilylation:
   Des groupes fonctionnels sont fixés à la surface de nanoparticules de silicium poreux à terminaisons Si-H par l'intermédiaire de liaisons Si-C selon la procédure enseignée dans la publication Buriak, J. M., Chem. Rev. 2002, 102 (5), 1272-1308. L'hydrosilylation est connue pour améliorer la stabilité chimique des structures de type silicium poreux dans des milieux aqueux. Des groupements fonctionnels isocyanate, amino ou semicarbazide sont attachés par hydrosilylation avec de l'allylamine, des dérivés allylisocyanate, ou des dérivés semicarbazide (protégés ou non) comme illustré sur la figure 6B, et sur la figure 22A. De façon analogue on peut à l'aide d'un l'alcénylamine, ou d'un alcénylisocyanate fonctionnaliser des nanoparticules de silicium poreux avec un groupement W de taille variable.

On obtient ainsi un silicium poreux fonctionnalisé 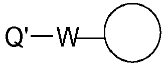
W a la même définition que dans la formule (I) et Q' représente un groupement choisi parmi : -NH₂, -N=C=O, -N=C=S, -NH-CO-NH-NH₂.

### □ Greffage des nanoparticules par une molécule photosensibilisante de type porphyrine.

On utilise des porphyrines fonctionnalisées par un groupement amino, isocyanate ou isothiocyanate répondant à la formule (II) ci-dessous, dans laquelle les paramètres x, M, X, R', R",Z⁺,Y⁻, A⁻, R1 ont la même définition que dans la formule (I), Q représente un groupement choisi parmi : -NH₂, -N=C=O, -N=C=S.

De telles molécules sont notamment décrites avec leur procédé de préparation dans J.M.Sutton et al., Bioconjugate chem. 2002, 13, 249-263 ; Yihui Chen et al., Bioconjugate chem. 2008, 19, 5-9.

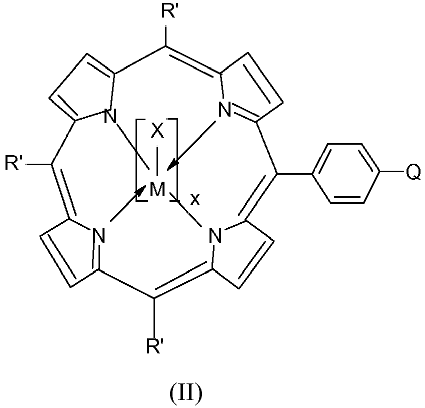

La synthèse de porphyrines porteuses de groupements pyridine est décrite notamment dans EP-345171 et dans WO2010/029232.

Lorsque la nanoparticule est greffée par des groupements amino, on utilise une porphyrine greffée par des isocyanates ou isothiocyanates. Lorsque la nanoparticule est greffée par des groupements isocyanates ou isothiocyanates, on utilise une porphyrine greffée par des amines. Le greffage des nanoparticules par la porphyrine se fait par formation d'un groupement urée ou thiourée.

### □ Greffage des nanoparticules par une molécule de ciblage.

Les molécules de ciblage sont fixées suivant plusieurs procédures illustrées sur la figure 9 déjà décrite pour les nanoparticules de silice mésoporeuses (Brevet, D. et al., Chem. Commun. 2009, 12, 1475-1477; Agemy, L., et al., Blood 2010, 116, 2847-2856), et sur la figure 22B.

De préférence les nanoparticules de l'invention sont greffées sur leur surface par des molécules de ciblage spécifiques des tissus néoplasiques, c'est-à-dire des biomolécules dont les récepteurs sont surexprimés par les cellules cancéreuses, ou à la surface des cellules cancéreuses. Ces molécules de ciblage facilitent le transfert des nanoparticules vers leur cible biologique. D'une façon générale, ces molécules de ciblage peuvent être choisies parmi : l'acide folique, les peptides, les hydrates de carbone, les anticorps. Elles peuvent être greffées sur les nanoparticules de l'invention par l'intermédiaire d'un linker polymérique.

Si les formulations présentent des propriétés de circulation réduites, des chaînes PEG peuvent être utilisées pour relier les nanoparticules à la fraction glucidique par couplage à l'aide de fonctionnalités squarate et amide ou semicarbazone. Le chitosan ou l'albumine sérique peuvent être utilisés pour modifier la chimie de surface des nanoparticules pour améliorer leur biocompatibilité. Le poids moléculaire et la longueur de chaîne de ces espèces peuvent être modulées pour optimiser la biocompatibilité, la pénétration cellulaire, et pour contrôler la cinétique de dégradation des nanostructures de silicium poreux.

Les peptides et/ou les agents de ciblage de cellules cancéreuses, peuvent être greffés sur la surface des nanoparticules de silicium poreux suivant des procédés similaires. Des peptides de ciblage peuvent être conjugués à des nanoparticules modifiées à base de silicium poreux par l'intermédiaire du groupe sulfhydryle cystéine en utilisant une allyle groupe de liaison PEG-MAL, qui peut être ajouté à la surface déjà modifiée de la nanoparticule poreuse de Si au moyen d'une d'hydrosilylation.

Selon une variante de l'invention, les molécules de ciblage et la porphyrine sont greffées au cours d'une seule étape.

### - Les utilisations des nanoparticules de l'invention:

Les nanoparticules de l'invention peuvent être utilisées à des fins thérapeutiques et/ou diagnostiques.

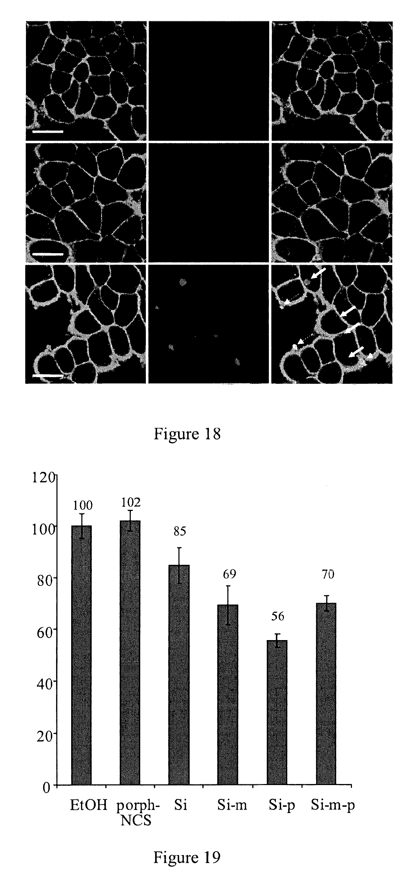

La production d'oxygène singulet et donc la destruction des cellules cancéreuses est induite par excitation photonique : Après irradiation des nanoparticules par une source lumineuse émettant dans l'infrarouge en mono- ou bi-photonique, l'oxygène singulet formé permet de détruire les cellules tumorales, comme illustré sur les figures 19, 31 et 32. L'irradiation est faite à une longueur d'onde comprise entre 650 nm et 900 nm. En bi-photo nique, l'irradiation est faite préférentiellement par trois à douze scans de 1.57s chacun à une puissance de 20 à 140 mW, avantageusement de 50 à 100mW, et encore mieux aux environs de 80 mW et pour une surface scannée de 1,5x1,5 mm2 soit une fluence de 10,6 J cm⁻² à 42,4 J cm-². En monophotonique, l'irradiation est faite préférentiellement par irradiation pendant 40 min à une puissance de 7 mW soit une fluence de 16.8 J/cm².

Notamment l'irradiation biphotonique de silicium poreux luminescent dans l'infra-rouge suivie du transfert d'énergie du silicium poreux aux molécules photosensibilisantes permet un traitement des cellules cibles efficace, par un mécanisme d'activation des molécules photosensibilisantes différent de celui mis en oeuvre par une irradiation monophotonique et présentant des possibilités de traitement moins invasifs pour le patient, avec moins d'effets secondaires que les traitements de l'art antérieur.

En outre, en raison des propriétés luminescentes du silicium poreux et la fluorescence de la porphyrine, ces nanoparticules permettent la visualisation par luminescence des tissus tumoraux ciblés et donc un suivi de l'efficacité du traitement, comme illustré sur les figures 18, 29, 30A et 30B. En outre, les porphyrines incorporées aux nanoparticules (I) sont fluorescentes si les cations métalliques M sont diamagnétiques ou si x = 0, aussi les nanoparticules de l'invention permettent aussi par ce moyen de procéder au marquage par fluorescence des zones où sont localisés les tissus cancéreux. Et certaines molécules de formule (I) comprennent un cation métallique paramagnétique (M dans la porphyrine) qui permet de suivre la répartition des nanoparticules dans l'organisme par RMN et IRJV1. Ces propriétés sont utiles aussi bien pour la détection d'une pathologie que pour le suivi de son traitement.

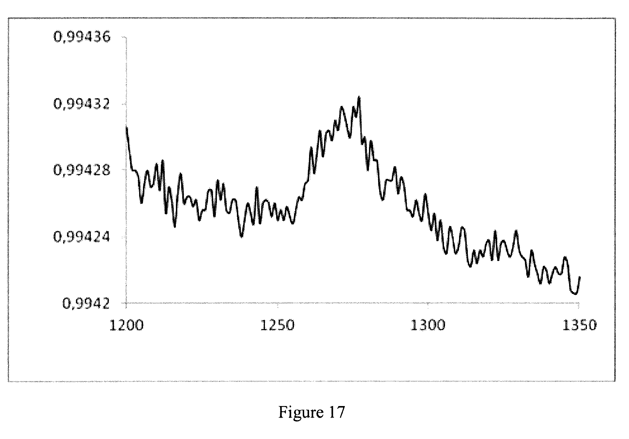

Les résultats obtenus montrent que les nanoparticules synthétisées sont efficaces pour générer de l'oxygène singulet en solution avec un rendement quantique cependant faible (figures 17, 23 et 24). Ces nanoparticules doivent permettre une vectorisation des actifs et leur endocytose dans les cellules tumorales après fonctionnalisation en surface par des biomolécules.

Parmi les différents cancers que l'on peut envisager de traiter avec les nanoparticules de l'invention, on peut citer : le rétinoblastome (lignées cellulaires Y-79), le cancer du colon (lignées cellulaires HT29), de l'épiderme (A 431), du poumon (A 549), du sein (MDA-MB-231, MCF-7), du col de l'utérus (HeLa) de l'ovaire (PEO 14), du mélanome (MDA-MB-435), de la prostate (LNCaP) ainsi que l'ensemble des tumeurs solides qui inclut, mais n'est pas limité aux cancers du cou et de la tête, aux cancers digestifs et des organes sexuels, et toute tumeur bénigne ou cancéreuse qui peut être illuminée. Le rayonnement infra-rouge pénétrant mieux dans l'organisme que le rayonnement visible, les nanoparticules de l'invention permettent d'atteindre de façon efficace des tissus cancéreux non accessibles par les traitements de l'art antérieur.

Après endocytose les lignées cellulaires sont irradiées, et l'efficacité des nanoparticules est évaluée par test MTT.

Les compositions de nanoparticules de l'invention peuvent être administrées localement ou par voie systémique. L'administration locale peut être effectuée notamment par injection de la composition de nanoparticules à proximité de la zone tumorale. Dans le cas des tumeurs superficielles, les compositions de nanoparticules peuvent être administrées par voie topique, sous une forme galénique appropriée (solution, suspension, pâte, patch). L'administration par voie générale peut être mise en oeuvre par voie intraveineuse, intramusculaire, sous-cutanée, intrapéritonéale ou rectale. De telles formulations et leur mode de mise en oeuvre sont bien connus de l'homme du métier. Le dosage de la composition en actif de formule (I) ou (Ia) est adapté en fonction du poids et de l'âge du patient, de la nature, de la localisation et du stade de développement de la tumeur, de la voie d'administration choisie et de la dose d'irradiation utilisée.

La composition peut comprendre tout autre actif connu pour le traitement des tumeurs et/ou de leurs symptômes. Elle comprend les composants classiques de la galénique adaptés au mode d'administration choisi. En particulier, elle peut être sous une forme galénique favorisant la vectorisation vers les tissus cibles.

Les nanoparticules de l'invention permettent le traitement de diverses maladies y compris les cancers, les tumeurs, les maladies prolifératives cellulaires, les maladies inflammatoires et les lésions tissulaires. Elles peuvent être utilisées notamment pour le traitement des maladies de la peau.

Les nanoparticules de l'invention peuvent être associées à un support cosmétiquement acceptable pour former une composition cosmétique. Notamment, elles peuvent être formulées sous forme de crème de soin, de patch cosmétique, de masque.

Ces nanoparticules peuvent être utilisées pour traiter, prévenir, retarder l'apparition et/ou la récidive d'une pathologie choisie parmi les cancers, les tumeurs, les maladies prolifératives cellulaires. Elles permettent également la détection de ces pathologies.

L'invention a encore pour objet un kit pour la détection, le traitement, la prévention, pour retarder l'apparition et/ou la récidive d'une pathologie choisie parmi les cancers, les tumeurs, les maladies prolifératives cellulaires comprenant :
- des nanoparticules de silicium poreux greffées de façon covalente par au moins une molécule photosensibilisante, ou une composition de médicament les comprenant
   et
- des moyens permettant une irradiation biphotonique dans l'infra-rouge.

Parmi les moyens utilisables pour irradier dans l'infra rouge en biphotonique, on peut mentionner un laser titane saphir femtoseconde focalisé.

En effet, les inventeurs ont mis en évidence pour la première fois la capacité des nanoparticules de silicium poreux, greffées de façon covalente par au moins une molécule photosensibilisante, à former des espèces oxydantes capables de détruire des cellules malignes sous l'effet d'une irradiation biphotonique dans le proche infra-rouge.

Selon cette variante, les molécules photosensibilisantes peuvent être de tout type, dès lors qu'elles sont greffées par l'intermédiaire d'une liaison covalente aux nanoparticules de silicium poreux. En particulier elles peuvent être choisies parmi les porphyrines, mais aussi parmi les chlorines ou les bactériochlorines Selon cette variante, de préférence les nanoparticules de silicium poreux sont également greffées par au moins une molécule de ciblage permettant de cibler spécifiquement des tissus néoplasiques.

L'invention a encore pour objet des nanoparticules de formule (I) ou (Ia) pour leur utilisation pour traiter prévenir, pour retarder l'apparition et/ou la récidive d'une pathologie choisie parmi les cancers, les tumeurs, les maladies prolifératives cellulaires comprenant : l'administration à un individu à traiter de nanoparticules de silicium poreux greffées par l'intermédiaire d'une liaison covalente par au moins une molécule photosensibilisante et le traitement par irradiation biphotonique dans le proche infra-rouge.

L'invention a encore pour objet un kit pour le traitement, la prévention et/ou la détection d'une pathologie choisie parmi les cancers, les tumeurs, les maladies prolifératives cellulaires comprenant des nanoparticules de formule (I) ou (Ia) ou des compositions pharmaceutiques les comprenant et des moyens permettant une irradiation dans l'infra-rouge, en particulier des moyens d'irradiation biphotoniques.

### - Figures :

Figure 1 : Représentation schématique de la préparation de nanoparticules de silicium par anodisation électrochimique de silicium cristallin, électrodissolution, et fracturation ultrasonique.
Figure 2 : Photographie d'imagerie SEM d'un film de silicium poreux, précurseur des nanoparticules, en surface (2A) et en coupe (2B).
Figure 3 : Photographie d'imagerie SEM (3A) et TEM (3B) des nanoparticules de silicium poreux préparées par fracturation ultrasonique et représentées sur la figure 1.
Figure 4 : Graphique représentant l'intensité de diffusion de la lumière en kcps en fonction de la taille des nanoparticules (diamètre hydrodynamique mesuré en nm).
Figure 5 : Graphique représentant l'isotherme d'adsorption et de désorption d'azote pour les nanoparticules de silicium poreux gravé à 200mA/cm². La quantité adsorbée (Volume adsorbé en mL STP/g) est représentée en fonction de la pression relative P/P₀.
Figure 6 : Représentation schématique de deux voies de synthèse permettant le greffage de groupes fonctionnels sur des nanoparticules de silicium poreux.
Figure 7 : Représentation schématique du couplage d'une porphyrine aminée sur une nanoparticule de silicium poreux fonctionnalisée par un groupement allylisocyanate.
Figure 8 : Représentation schématique du couplage d'une porphyrine fonctionnalisée par un isocyanate sur une nanoparticule de silicium poreux fonctionnalisée par un groupement allylamine.
Figure 9 : Représentation schématique du couplage d'un groupement mannose sur une nanoparticule de silicium poreux par l'intermédiaire d'un groupement squarate.
Figure 10A : Graphique représentant le spectre FTIR (nombre d'onde en cm⁻¹) des nanoparticules de silicium poreux non greffées (continu) et hydrosilylées avec l'allylamine (pointillé). Figure 10B : Graphique représentant le spectre FTIR (nombre d'onde en cm⁻¹) de nanoparticules de silicium poreux à différentes étapes du couplage d'un mannose-squarate : après hydrosilylation par une allylamine (continu), après couplage avec le mannose (pointillé).
Figure 11 : Graphique représentant le spectre UV-VIS d'une solution obtenue après dissolution dans KOH des nanoparticules de silicium poreux greffées avec la porphyrine-NCS, avec 74 µg de porphyrine/mg de nanoparticules, soit un chargement de 7,4% en masse (absorbance Log I/I0 en fonction de la longueur d'onde en nm).
Figure 12 : Graphique représentant le spectre FTIR (nombre d'onde en cm⁻¹) de nanoparticules de silicium poreux à différentes étapes du couplage d'une porphyrine-NCS: après hydrosilylation par une allylamine (continu), après couplage avec la porphyrine-NCS (pointillé).
Figure 13 : Graphique représentant le spectre FTIR (nombre d'onde en cm⁻¹) de nanoparticules de silicium poreux greffées avec une porphyrine-NH₂ par la voie allylisocyanate.
Figure 14A: Graphique représentant le spectre FTIR (nombre d'onde en cm⁻¹) de nanoparticules de silicium poreux à différentes étapes du couplage simultané d'un mannose-cétone et d'une porphyrine-NCS: (a) silicium poreux greffé au semicarbazide, (b) après hydrosilylation par une allylamine, (c) après couplage avec la porphyrine-NCS.
Figure 14B: Graphique représentant le spectre UV-VIS d'une solution obtenue après dissolution dans KOH des nanoparticules de silicium poreux greffées avec la porphyrine-NCS, et avec le semicarbazide, avec 10 µg de porphyrine/mg de nanoparticules (absorbance Log I/I0 en fonction de la longueur d'onde en nm).
Figure 15 : Graphique représentant la quantité produite d'oxygène singulet en fonction de la longueur d'onde d'irradiation pour des nanoparticules sans porphyrine (intensité exprimée en unité arbitraire : phosphorescence de l'oxygène singulet en fonction de la longueur d'onde en nm).
Figure 16 : Graphique représentant le spectre UV-VIS des nanoparticules de silicium poreux greffées par une porphyrine et par du mannose (absorbance en Log I/I0 en fonction de la longueur d'onde en nm).
Figure 17 : Graphique représentant la quantité produite d'oxygène singulet en fonction de la longueur d'onde d'irradiation pour des nanoparticules de silicium poreux greffées par une porphyrine et par du mannose (intensité en exprimée en unité arbitraire : phosphorescence de l'oxygène singulet en fonction de la longueur d'onde en nm).
Figure 18 : Images par microscopie confocale de cellules vivantes MCF-7 du cancer du sein. Les cellules ont été incubées 5 h avec 20 pg ml-1-pSiNP Porph-NH₂ nanoparticules, 0,25 µg ml-1 Porph-NH₂, ou un véhicule (éthanol) et co-colorées avec un marqueur membranaire (masque de cellule). Les membranes cellulaires ont été visualisées en vert à 561 nm, les nanoparticules et les porphyrines ont été visualisées à 633 nm et les images ont été fusionnées. Les photos sont représentatives d'au moins 3 expériences indépendantes. Les agrégats intracellulaires de pSiNP-Porph-NH₂ sont identifiés par des flèches "solides" et les agrégats de la membrane cellulaire, qui apparaissent en jaune dans l'image de fusion, sont marqués par des «flèches en pointillés". Les barres d'échelle sont à 5 µm
   Colonne gauche : membrane, colonne du milieu : composés, colonne droite fusion.
   Première ligne : contrôle, seconde ligne : Porphyrine-NH₂, troisième ligne : nanoparticules de silicium poreux greffées à laPorphyrine-NH₂.
Figure 19 : Graphique représentant le % de cellules MCF7 vivantes en fonction du traitement qui leur a été administré. Efficacité de la PDT *in vitro* en excitation biphotonique sur les cellules MCF-7 incubées avec : du milieu de culture contrôle contenant 4% d'éthanol (EtOH, expérience de contrôle), de la porphyrine-NCS libre (porph-NCS), des nanoparticules de silicium poreux non greffées (Si), des nanoparticules de silicium poreux greffées au mannose (Si-m), des nanoparticules de silicium poreux greffées à la porphyrine-NCS (Si-p) et des nanoparticules de silicium poreux greffées à la porphyrine et au mannose (Si-m-p). Irradiation à 750 nm, 3 scans de 1,57 s. Le décompte des cellules vivantes est réalisé par un test MTS 2 jours après irradiation.

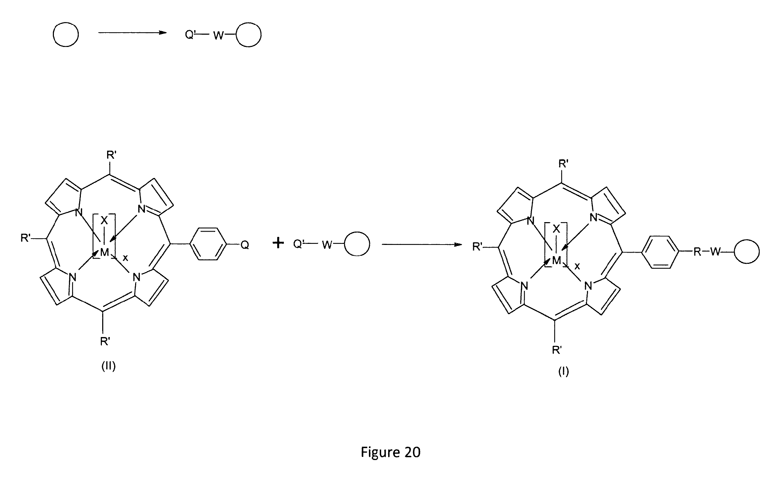

Figure 20 : Représentation schématique de la préparation des nanoparticules greffées avec une porphyrine.
Figure 21 : Diffractogrammes de rayons X des nanoparticules de silicium poreux : Figure 21A aux grands angles, Figure 21B aux petits angles. L'intensité relative (u.a.) est représentée en fonction de 2θ (en degrés)
Figure 22A : Représentation schématique du couplage d'un groupement semicarbazide sur une nanoparticule de silicium poreux par hydrosilylation.
Figure 22B : Représentation schématique du couplage d'un groupement mannose sur une nanoparticule de silicium poreux par l'intermédiaire d'une fonction semi-carbazone.
Figure 23 : Courbes traçant ln(A) en fonction du temps d'illumination (minutes) pour le bleu de méthylène (losange), la porphyrine-NCS (triangle) et les nanoparticules de silicium poreux greffées avec la porphyrine-NCS (carré).
Figure 24 : Courbes traçant l'absorbance à 411 nm ln(A) en fonction du temps d'illumination (minutes) pour le DPBF seul (carré), et pour les nanoparticules de silicium poreux greffées avec la porphyrine-NH₂ + DPBF (losange).
Figure 25 : Cinétique du relargage de la porphyrine-NCS à partir des nanoparticules de silicium poreux greffées à la porphyrine-NCS incubées à 37°C dans différents milieux. Losange: dans le PBS à pH = 7,4, carré: dans le milieu de culture DMEM à 10% de FBS, triangle: dans le milieu de culture DMEM sans sérum.

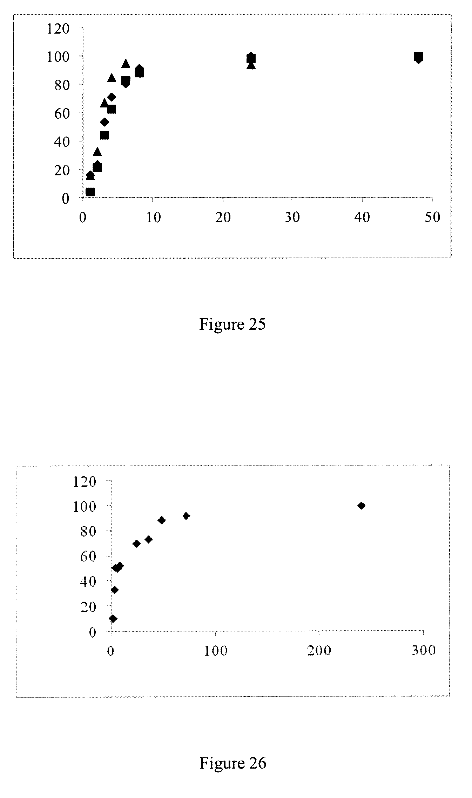

Figure 26 : Cinétique de libération de la porphyrine-NH₂ des nanoparticules greffées à la porphyrine-NH₂, incubées à 37 ° C dans du PBS (pH = 7,4). La stabilité des nanoparticules de silicium poreux greffées à la porphyrine-NH₂ dans du PBS a été suivie par la détermination spectroscopique de la quantité de porphyrine apparaissant dans la solution en fonction du temps (heures). Les données indiquent que 50% de la porphyrine a été libéré au bout de 5 h, et que 89% de la porphyrine a été libéré après 48h d'incubation. Dans les conditions expérimentales utilisées dans cette étude, ce temps de dégradation est compatible avec l'utilisation des nanoparticules de silicium poreux greffées à la porphyrine-NH₂ pour l'imagerie et la PDT.
Figure 27: Quantification de la porphyrine-NH₂ contenue dans les nanoparticules de silicium poreux (noir) et de la porphyrine libre (gris) internalisée dans les cellules vivantes du cancer du sein MCF-7 après 5 h d'incubation. L'internalisation a été suivie par une détermination spectroscopique de la quantité de porphyrine dans les lysats cellulaires et comparée à une série de dilutions. La quantité de porphyrine internalisée à partir des nanoparticules est dans l'intervalle de 105 ± 5 ng.ml-1 de porphyrines tandis que la porphyrine libre internalisée correspond à 35 ± 5 ng.ml-1.
Figure 28: Analyse semi-quantitative de nanoparticules greffées à la porphyrine-NH₂ (pSiNP-Porph-NH₂) et de la porphyrine libre (Porph-NH₂) internalisées dans les cellules vivantes du cancer du sein MCF-7 après 5 h d'incubation. Une intensité moyenne à l'intérieur des cellules a été déterminée à partir de cinq zones distinctes et exprimée en valeur de gris par µm². On a constaté que les nanoparticules internalisées représentent 45% du total des nanoparticules détectées.

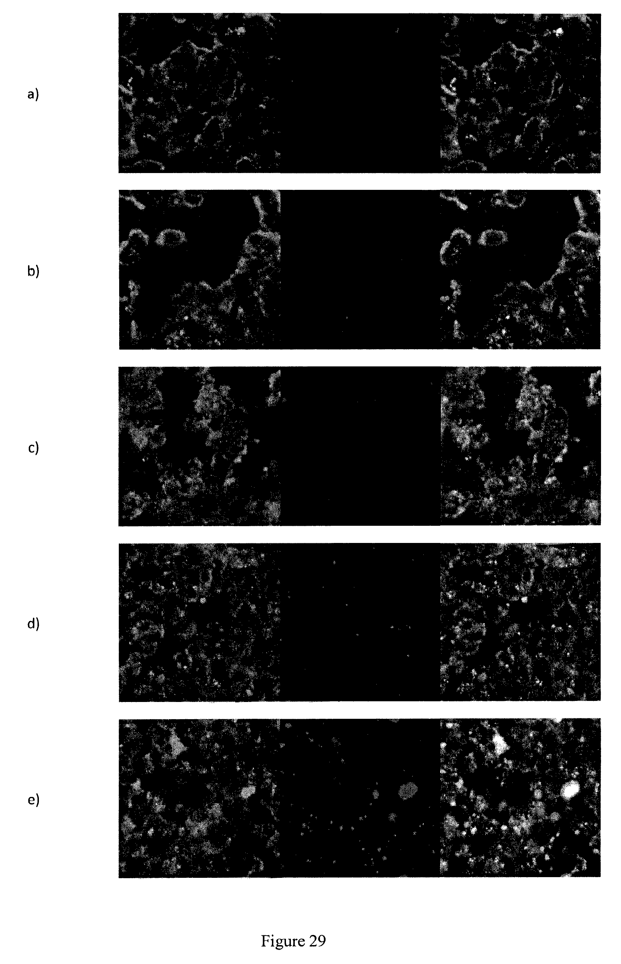

Figure 29 : Images de microscopie confocale en excitation monophotonique à 405 nm de cellules MCF-7 incubées avec du milieu contrôle, des pSiNP (nanoparticules de silicium poreux) non greffées, de la porphyrine-NCS libre, des pSiNP-porphyrine-NCS et des pSiNP-mannose-porphyrine-NCS. A gauche : Autofluorescence des cellules (émission récoltée entre 540 nm et 620 nm), au milieu : nanoparticules et porphyrine (émission récoltée entre 620 nm et 700 nm), à droite : superposition de deux images de fluorescence. a) contrôle, b) pSiNP c) Porphyrine-NCS libre d) pSiNP-porphyrine-NCS e) pSiNP-mannose-porphyrine-NCS

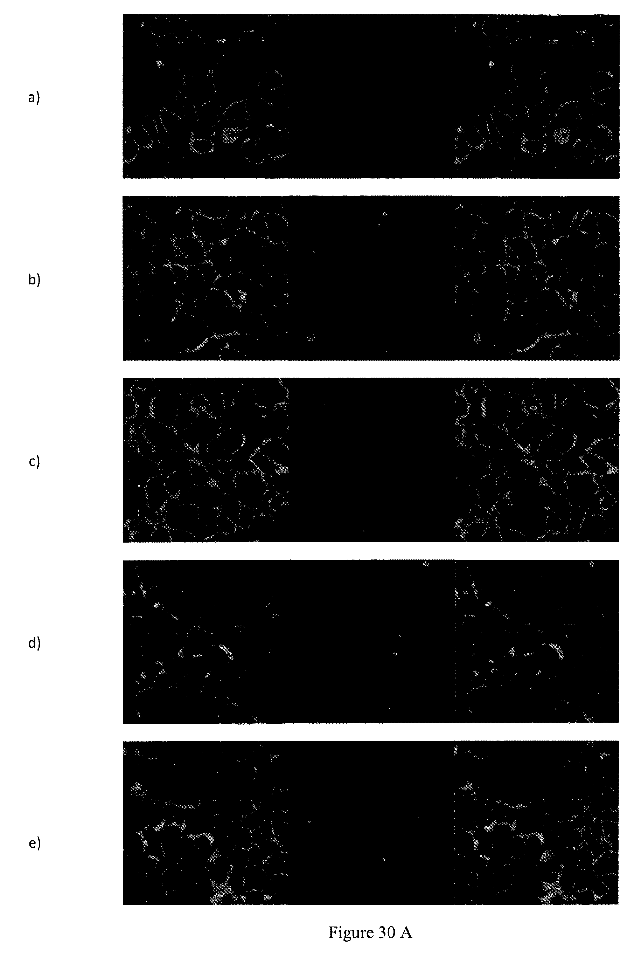

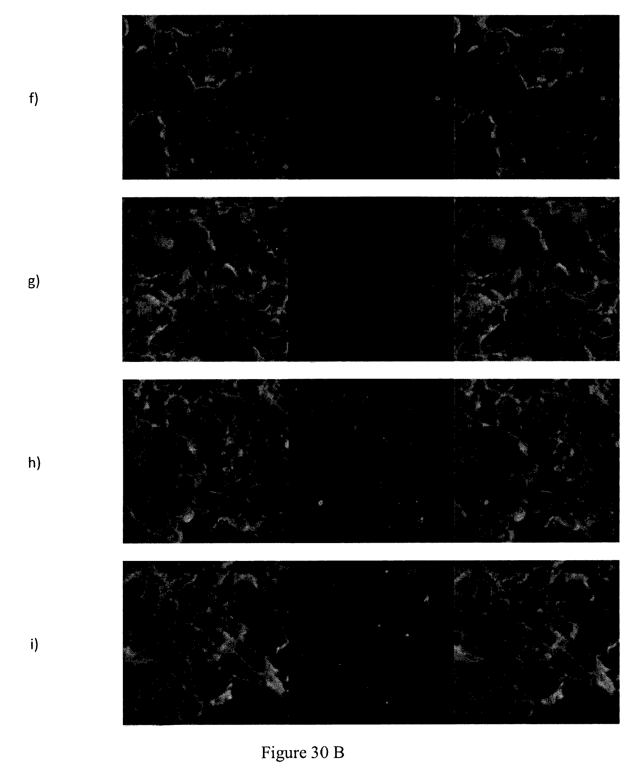

Figures 30A et 30B: Imagerie en microscopie confocale multiphotonique de cellules MCF-7 incubées avec les différents types de nanoparticules de silicium poreux. Les images ont été prises avec un détecteur spectral durant une excitation biphotonique à 750 nm. A gauche : fluorescence du marqueur membranaire (Cell Mask Orange), au milieu : fluorescence émise entre 620 et 700 nm, à droite : superposition des deux images de fluorescence. a) Ethanol b) pSiNP (nanoparticules de silicium poreux) c) Porphyrine-NCS libre d) pSiNP + porphyrine libre e) pSiNP-porphyrine-NCS f) pSiNP-mannose g) pSiNP-mannose + mannose libre h) pSiNP-mannose-porphyrine-NCS i) pSiNP-mannose-porphyrine-NCS + mannose libre
Figure 31. Efficacité de la PDT *in vitro* en excitation monophotonique, en pourcentage de cellules vivantes, avec la porphyrine-NH₂ libre à 0,25 µg/mL, les pSiNP (nanoparticules de silicium poreux) non greffées à 20 µg/mL et les pSiNP-porphyrine-NH₂ à 20 µg/mL, avec ou sans irradiation. Irradiation à 650 nm (14 J/cm²) pendant 40 min. Le décompte des cellules vivantes est réalisé par un test MTT 2 jours après irradiation.

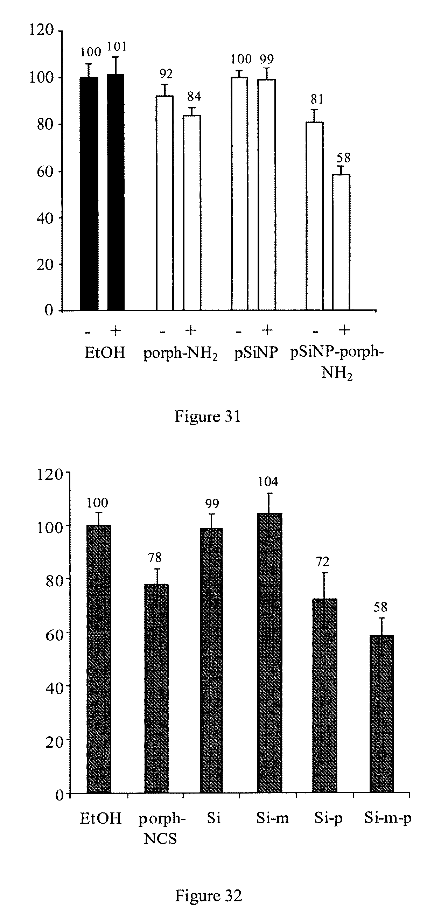

Figure 32. Efficacité de la PDT *in vitro* en excitation monophotonique, en pourcentage de cellules vivantes, sur les cellules MCF-7 incubées avec : du milieu de culture contrôle contenant 4% d'éthanol (EtOH, expérience de contrôle), de la porphyrine-NCS libre (porph-NCS), des nanoparticules de silicium poreux non greffées (Si), des nanoparticules de silicium poreux greffées au mannose (Si-m), des nanoparticules de silicium poreux greffées à la porphyrine-NCS (Si-p) et des nanoparticules de silicium poreux greffées à la porphyrine et au mannose (Si-m-p). Irradiation à 650 nm, 7mW/cm², 40 min. Le décompte des cellules vivantes est réalisé par un test MTS 2 jours après irradiation.
Figure 33. Pointillé : spectre d'absorption UV-VIS normalisé de la porphyrine-NCS. Continu : spectre normalisé d'émission des nanoparticules de silicium poreux dans l'eau (excitation à 250 nm).

### - Partie expérimentale:

### 1. Synthèse des nanoparticules de silicium poreux biodégradables et fonctionnalisation chimique.

Cette partie décrit la préparation des composants nanostructurés / nanoparticules et le développement de chimies de greffage originales pour l'attachement des porphyrines et des agents de ciblage, pour l'étude de leur efficacité à cibler, pénétrer et délivrer les agents thérapeutiques de type porphyrine dans des cellules cancéreuses ou endothelial *in vivo.*

### 1.1. Synthèse et caractérisation des nanostructures/nanoparticules de silicium poreux

Les nanoparticules de silicium poreux sont préparées par gravure électrochimique de plaques de silicium monocristallin dans une solution électrolytique d'acide fluorhydrique (HF) dans l'éthanol, suivie d'un décollement de la couche poreuse par électrodissolution et d'une fracture par ultrasons d'après une procédure publiée (figure 1), (Sailor, M. J. et al., Adv. Funct. Mater. 2009, 19 (20), 3195-3208 ; Bley, R. A. et al. Chem. Mat. 1996, 8 (8), 1881-1888).

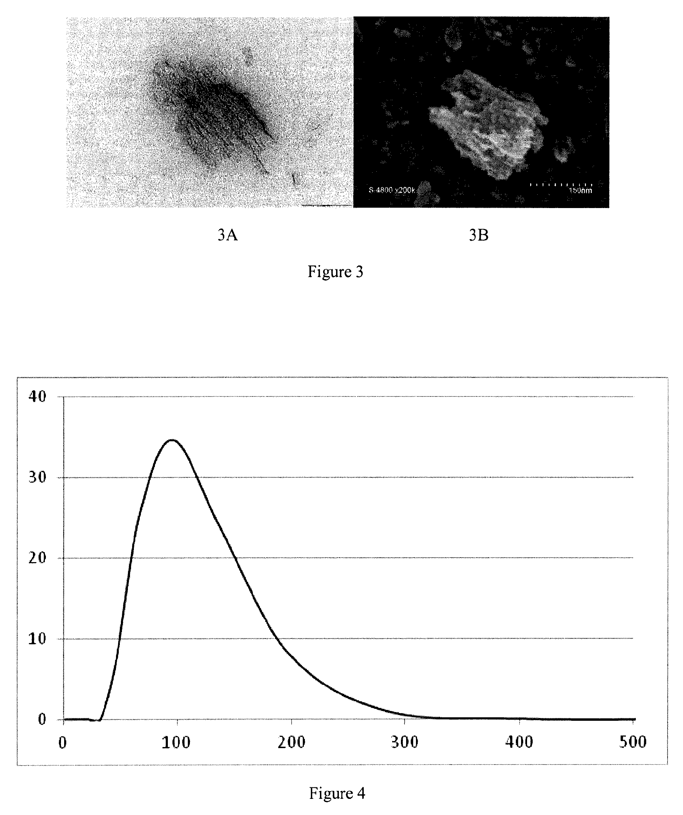

Du silicium monocristallin de type p++ (dopé au bore, résistivité 0.8-1.2mΩ.cm, orientation, <100>) est gravé par voie électrochimique dans une solution d'acide fluorhydrique (48%)/éthanol à 3:1 en volume (figure 1, première étape). La gravure est réalisée dans une cellule en téflon, en utilisant une contre-électrode circulaire de platine. Un courant constant de 200mA/cm² est appliqué pendant 150 secondes, et l'échantillon est rincé trois fois à l'éthanol. La couche poreuse est ensuite électro-dissoute dans une solution d'HF dans l'éthanol à 3.3% en appliquant un courant constant de 4 mA/cm pendant 250 secondes (étape 2 de la figure 1). Après trois rinçages à l'éthanol, la couche poreuse est déposée dans l'éthanol, dans un flacon en verre. L'échantillon, après avoir été dégazé sous flux d'azote pendant 20 minutes, est soniqué dans un bain à ultrasons pendant 16 heures (étapes 3 et 4 de la figure 1). La dispersion obtenue de particules dans l'éthanol est filtrée (0,2 µm nylon syringe filter) et centrifugée à 14000 rpm pendant 30 minutes. Des nanoparticules d'une distribution de taille centrée à 115 nm sont obtenues (fig.4). La taille et la texture des nanoparticules sont caractérisées par microscopie électronique à balayage (MEB), microscopie électronique à transmission (MET), diffusion de lumière (DLS) et adsorption d'azote (BET). Les nanoparticules sont aussi caractérisées par diffraction des rayons X.

On observe sur la figure 2 l'image MEB d'une surface et d'une section d'un film de Si poreux.

On observe sur la figure 3 les images MEB et MET de nanoparticules de Si poreux préparées par fracture ultrasonique du film poreux présenté sur la figure 2.

La figure 4 illustre la courbe de diffusion de lumière pour les nanoparticules de Si poreux présentées sur la figure 3.

La figure 5 représente la courbe isotherme d'adsorption/désorption d'azote pour les nanoparticules de Si poreux présentées sur la figure 3.

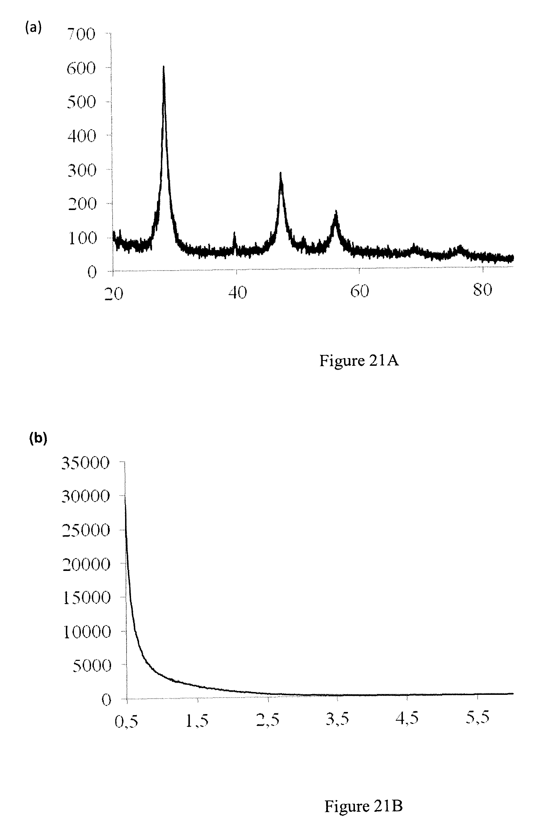

Les figure 21A et 21B présentent les diffractogrammes de rayons X sur poudre aux grands angles et aux petits angles pour les nanoparticules de Si poreux présentées sur la figure 3.

### 1.2. Modification de la surface des nanostructures/nanoparticules de silicium poreux

Des porphyrines synthétiques hydrophiles sont ancrées de manière covalente à la surface des nanostructures/nanoparticules de silicium poreux par des chimies de greffage originales. Des espèces ciblantes sont également greffées à la surface des nanostructures/nanoparticules de silicium poreux. En amont du greffage des espèces ciblantes et des porphyrines, les nanostructures/nanoparticules de silicium poreux sont modifiées chimiquement par des ligands fonctionnels

### 1.2.1. Modification chimique des nanostructures/nanoparticules de silicium poreux par des ligands fonctionnels :

Les nanostructures/nanoparticules de silicium poreux fraîchement gravées, présentant des liaisons Si-H à leur surface sont très hydrophobes. Deux modifications de surface sont développées.

### 1.2.1.1. Oxydation controlée (thermale, ozone, diméthyl sulfoxide, borate aqueux) et silanisation :

Des espèces Si-OH et SiO₂ sont produites à la surface des nanostructures/nanoparticules. La chimie de silanisation sur des surfaces oxydées de nanostructures/nanoparticles de silicium poreux est développée pour le greffage de chaines carbonnées fonctionnelles telles que l'aminopropyl triethoxysilane, incorporant des groupements amines (comme illustré sur la figure 6A) utilisés ensuite pour le greffage de porphyrines et d'espèces ciblantes.

Après oxidation des nanoparticules, elles sont redispersées dans l'aminopropyl triéthoxysilane. La réaction de silanisation est réalisée à 150°C sous flux d'azote pendant 2 heures. Les nanoparticules sont ensuite rincées 4 fois à l'éthanol et deux fois au diéthyléther, avant d'être séchées sous courant d'azote.

### 1.2.1.2. Hydrosilylation:

Des groupes fonctionnels sont greffés de manière covalente à la surface des nanostructures/nanoparticules de Si poreux via des liaisons Si-C selon des procédures publiées (Buriak, J. M., Chem. Rev. 2002, 102 (5), 1272-1308). L'hydrosilylation est un traitement chimique connu pour renforcer la stabilité chimique du silicium poreux en milieu aqueux (15). Des groupes fonctionnels de type amine, isocyanate, et semicarbazide sont ancrés par hydrosilylation avec des dérivés allylamines, allylisocyanates, et allylsemicarbazide comme illustré sur la figure 6B et sur la figure 22A.

### 1.2.2. Greffage des porphyrines

Des porphyrines aminées et isocyanatées sont greffées aux différents types de ligands (amino- et isocyanato-) précédement ancrés à la surface ces nanoparticules de Si poreux.

### 1.2.2.1. Greffage de la porphyrine -NH₂ sur les nanoparticules de Si poreux hydrosilylées avec l'allylisocyanate.

Les nanoparticules sont dispersées dans l'allylisocyanate. L'hydrosilylation est réalisée à 90°C sous flux d'azote pendant 3 heures. Les particules sont ensuite rincées trois fois dans du tétrahydrofurane (THF) et redispersées dans 4 ml de THF.

500 µL de porphyrine -NH₂ à 1mg/mL dans du dimethylformamide (DMF) sont ajoutés à 200 µL d'éthanol. La réaction a lieu à 80°C sous reflux d'azote pendant 4 heures. Les particules sont ensuite rincées deux fois à l'éthanol, deux fois à l'eau, deux fois à l'éthanol, et deux fois au diéthyl ether avant d'être séchées sous courant d'azote.

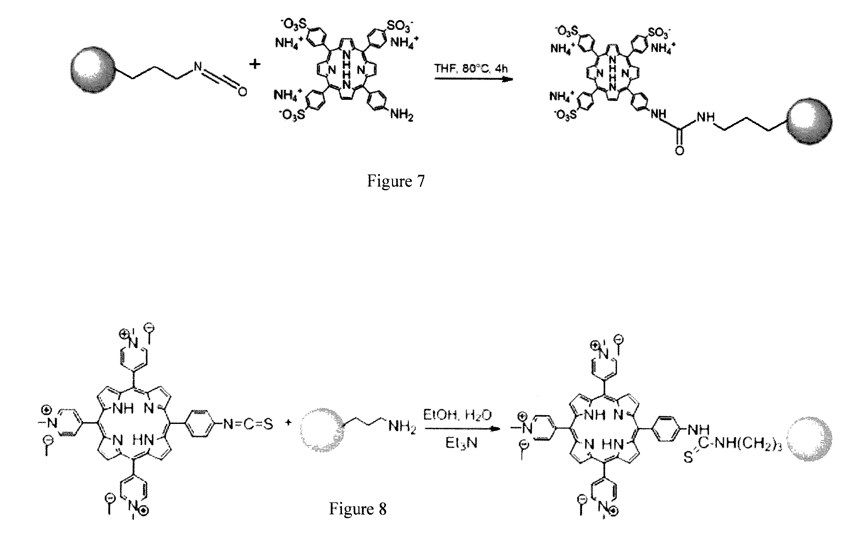

Cette procédure de greffage illustrée sur la figure 7 n'a jamais été décrite auparavant sur des nanoparticules de Si poreux.

### 1.2.2.2. Greffage de la porphyrine -NCS sur les nanoparticules de Si poreux hydrosilylées avec l'allylamine (illustrée sur la figure 8) :

Les nanoparticules sont dispersées dans l'allylamine. Cette solution est ensuite dégazée par trois cycles consécutifs de congélation-pompage-décongélation (freeze-pump-thaw cycles).

L'hydrosilylation est réalisée à 70°C sous flux d'azote pendant 2 heures et les nanoparticules sont rincées quatre fois dans de l'éthanol. Les particules sont redispersées dans 2 ml d'éthanol. 2.5 mL d'une solution d'H₂O/éthanol 1:1 (en volume) sont ensuite ajoutés à 500 µL d'une solution de porphyrine -NCS à 1 mg/mL dans l'éthanol.

La réaction a lieu à température ambiante sous agitation pendant 18 heures. Les particules sont ensuite rincées quatre fois à l'éthanol, et deux fois au diéthyl ether avant d'être séchées sous courant d'azote.

### 1.2.3. Ancrage des espèces ciblantes

Des molécules de type carbohydrate (mannose) sont couplées à la surface des nanoparticules de Si poreux selon une procédure développée précédemment pour des nanoparticules mésoporeuses de silice et illustrée sur la figure 9, et selon une autre procédure illustrée sur la figure 22B. Nous avons montré que des groupements squarate et semicarbazide permettent le couplage de molécules de type carbohydrate dans des conditions douces. Le rôle attendu des espèces carbohydrate est la vectorisation des nanoparticules sur les cellules cancéreuses.

### 1.2.3.1 Ancrage d'un mannose-squarate

Dans une réaction typique, les nanoparticules de Si poreux sont dispersées dans de l'allylamine. Cette suspension est ensuite dégazée par trois cycles consécutifs de congélation-pompage-décongélation.

L'hydrosilylation est réalisée à 70°C sous reflux d'azote pendant 2 heures et les nanoparticules sont rincées quatre fois dans de l'éthanol. Les particules sont redispersées dans 2,5 ml d'éthanol. 2.5 mL d'une solution de mannose-squarate à 6 mg/mL dans H₂O/éthanol 1:1 (en volume) sont ensuite ajoutés à 250 µL de triéthylamine.

La réaction a lieu à température ambiante sous agitation pendant 18 heures. Les nanoparticules sont ensuite rincées quatre fois au diéthyl ether avant d'être séchées sous courant d'azote.

### 1.2.3.2 Ancrage d'un mannose-cétone

### - Hydrosilylation des nanoparticules de silicium poreux avec le semi-carbazide :

Les nanoparticules de silicium poreux dispersées dans l'éthanol sont dans un premier temps centrifugées à 22000 g pendant 30 min, puis rincées 2 fois avec du THF avec centrifugation à 22000 g pendant 30 min à chaque fois. Les nanoparticules sont alors redispersées dans 3 mL d'une solution de tert-butyl-2-[(allylamino)carbonyl]hydrazine-carboxylate, à 10⁻¹ M dans le THF. La réaction d'hydrosilylation a alors lieu à 85°C pendant 3 h sous agitation et sous flux d'azote. Les particules sont ensuite centrifugées et rincées deux fois au THF, une fois à l'éthanol et une fois au dichlorométhane.

### - Déprotection de la fonction semi-carbazide

Afin d'enlever le groupement Boc protégeant la fonction semi-carbazide, les nanoparticules de silicium poreux sont centrifugées à 22000 g pendant 30 min, pour ensuite être redispersées dans 5 mL d'une solution d'acide trifluoroacétique (TFA) à 40% en volume dans du dichlorométhane. Cette dispersion est agitée pendant 4 h à température ambiante, puis les nanoparticules sont rincées deux fois au CH₂Cl₂, une fois à l'éthanol, une fois à l'eau, une fois à l'éthanol et une fois au CH₂Cl₂.

### - Greffage du mannose-cétone

Suite à la déprotection du semi-carbazide, les nanoparticules sont redispersées dans 2,5 mL d'éthanol. On ajoute alors au goutte à goutte 2,5 mL d'une solution de mannose-cetone à 1,6.10⁻² M dans un mélange eau/éthanol 1:1 et 250 µL de triéthylamine. La réaction a lieu sous agitation et à température ambiante pendant 18 h. Après la réaction, 4 à 5 lavages sont effectués à l'eau distillée puis deux lavages à l'éthanol absolu et deux autres lavages au diéthyléther. Enfin les particules greffées sont séchées sous azote.

### 1.2.4. Greffage simultané du mannose-squarate et de la porphyrine-NCS:

Les nanoparticules de Si poreux sont dispersées dans de l'allylamine. Cette suspension est ensuite dégazée par trois cycles consécutifs de congélation-pompage-décongélation. L'hydrosilylation est réalisée à 70°C sous reflux d'azote pendant 2 heures et les nanoparticules sont rincées quatre fois dans de l'éthanol. Les particules sont redispersées dans 2 ml d'éthanol. 2,5 mL d'une solution de mannose-squarate à 6 mg/mL dans H₂O/éthanol 1:1 (en volume) sont ensuite ajoutés à 250 µL de triéthylamine, et à 500 µL d'une solution de porphyrine -NCS à 1 mg/mL dans l'éthanol. La réaction a lieu à température ambiante sous agitation pendant 18 heures. Les nanoparticules sont ensuite rincées quatre fois à l'éthanol, et deux fois au diéthyl éther avant d'être séchées sous courant d'azote.

### 1.2.5. Greffage simultané d'un mannose-cétone et de la porphyrine NCS :

- Hydrosilylation des nanoparticules de silicium poreux avec le semi-carbazide (comme ci-dessus)
- Hydrosilylation des nanoparticules de silicium poreux avec l'allylamine (comme ci-dessus)
- Greffage de la porphyrine-NCS (comme ci-dessus)
- Greffage du mannose-cétone (comme ci-dessus)

### Remarques :

1/ Le galactose peut être substitué au mannose, en suivant les mêmes conditions expérimentales.
2/ Si les formulations de nanoparticules présentent de faibles propriétés de circulation, des chaînes de PEG (polyethylène glycol) peuvent être utilisées pour lier les espèces carbohydrate aux nanoparticules via des couplages squarate ou amide.

Le chitosan et le sérum albumine peuvent être utilisés comme autre chimie de surface alternative pour améliorer la biocompatibilité des nanoparticules. Le poids moléculaire et la longueur de chaîne des PEGs pourra être ajusté pour maximiser la biocompatibilité, la pénétration cellulaire, et pour contrôler les cinétiques de dégradation des nanostructures de silicium poreux.

### 1.2.6. Caractérisation

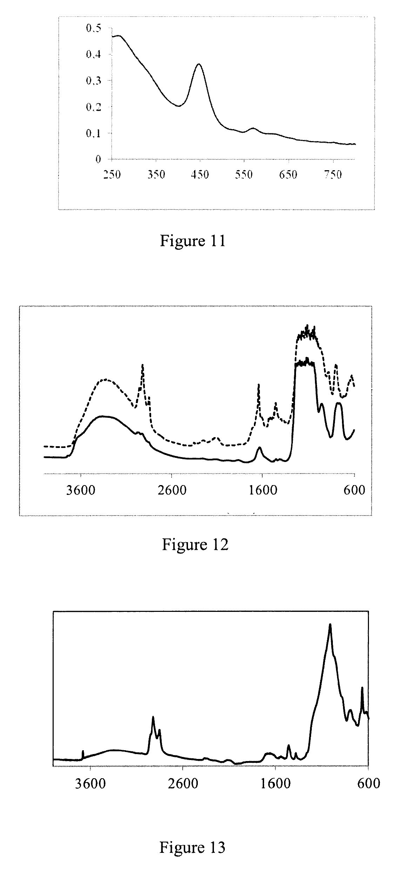

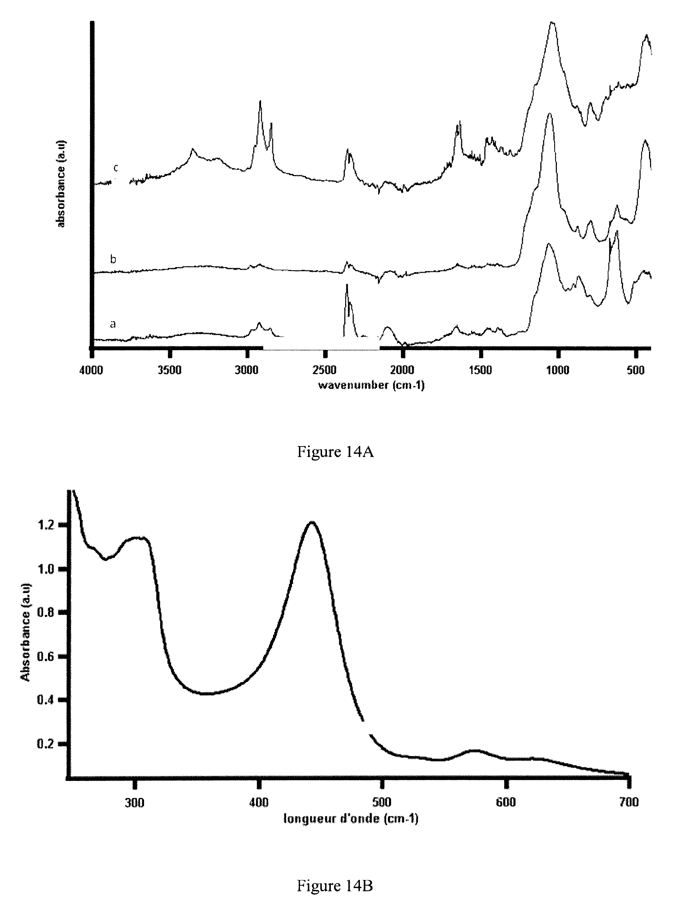

Les nanoparticules modifiées chimiquement sont caractérisées par spectroscopie de vibration Infra-rouge (FTIR), analyse élémentaire, et XPS pour le greffage du mannose, et par spectroscopie d'absorption UV-VIS et FTIR pour le greffage des différentes porphyrines à la surface des nanoparticules de Si poreux. Pour la spectroscopie d'absorption UV-VIS les nanoparticules de Si poreux sont dissoutes dans une solution de KOH avant de mesurer l'absorbance de la porphyrine (figures 11 et 14B).

### 1.2.6.1. Greffage du mannose

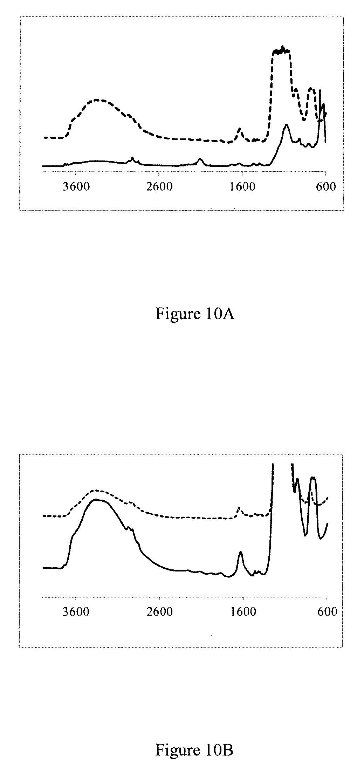

Le greffage du mannose squarate sur les nanoparticules fonctionnalisées à l'allylamnie se fait par addition nucléophile de l'amine suivie par l'élimination du groupe éthoxy du squarate. L'électrophilie du squarate est augmentée par la présence de liaisons hydrogène avec l'eau, co-solvant de la réaction. La triéthylamine catalyse la réaction en captant les protons libérés lors de la réaction. Le spectre FTIR des nanoparticules fonctionnalisées au mannose est présenté sur la figure 10B.

Sur ce spectre FTIR, la bande apparaissant à 1654 cm⁻¹ est attribuée à la vibration de déformation angulaire de la liaison N-H des amines ayant réagi. La bande de vibration à 1630 cm⁻¹ est toujours présente sous la forme d'un épaulement dans la bande centrée à 1654 cm⁻¹. La présence de cette bande, attribuée à la déformation angulaire de la liaison N-H de l'amine primaire, signifie que l'amine primaire est en partie toujours présente à la surface des nanoparticules de silicium poreux.

Les résultats des analyses élémentaires après greffage du mannose indiquent ci-dessous le pourcentage d'éléments présents:
- 5.25%ofC
- 0.72% of N
- 4.18% of O

Ces valeurs sont cohérentes avec la présence de mannose à la surface des particules de Si poreux.

### Quantification par XPS

Experimental : Les analyses XPS ont été effectuées sur un appareil ESCALAB 250 de Thermo Electron du plateau technique d'analyse de l'Université Montpellier 2. La source d'excitation est la raie Al Kα (1486,6 eV). La surface analysée a un diamètre de 400 µm, les spectres de photoélectrons sont calibrés en énergie de liaison par rapport à l'énergie de la composante C-C du carbone C1s à 284,8 eV, et la charge est compensée par un faisceau d'électrons.

**Tableau 1 : Tableau présentant les quantifications obtenues par XPS pour les nanoparticules de silicium poreux greffées avec le mannose-squarate (sur l'allylamine).**

| **Name** | **Peak BE** | **Height Counts** | **FWHM eV** | **Area (P) CPS.eV** | **Area (N)** | **At. %** |
|---|---|---|---|---|---|---|
| Si2p | 103,22 | 10204,45 | 1,78 | 19956,09 | 0,56 | 30,98 |
| C1s | 284,89 | 3324,17 | 2,73 | 9935,02 | 0,23 | 12,76 |
| N1s | 401,07 | 400,97 | 3,81 | 1551,23 | 0,02 | 1,12 |
| O1s | 532,53 | 65969,95 | 1,62 | 118334,22 | 0,96 | 53,42 |
| F1s | 688,97 | 2728,87 | 1,83 | 5593,67 | 0,03 | 1,72 |

### 1.2.6.2 Greffage des porphyrines

Le spectre UV-VIS d'une solution obtenue après dissolution des nanoparticules de silicium poreux greffées avec la porphyrine-NCS dans KOH est présenté sur la figure 11. 74 µg de porphyrine/mg de nanoparticules, soit un chargement de 7,4% en masse sont obtenus.

Le spectre FTIR des nanoparticules de silicium poreux greffées avec une porphyrine-NCS est présenté sur la figure 12. On observe trois bandes intenses entre 2850 et 2960 cm⁻¹, correspondant aux vibrations d'élongation des liaisons C-H. Ceci est dû aux nombreuses liaisons C-H apportées par la porphyrine. Entre 1460 cm⁻¹ et 1600 cm⁻¹, le spectre vibrationnel est complexe. Ces bandes sont caractéristiques des liaisons C=C aromatiques présentes grâce à la porphyrine. La bande intense observée à 1642 cm⁻¹ pourrait être caractéristique de la liaison thiourée ou bien de vibration de déformation angulaire de N-H des amines n'ayant pas réagi. Enfin la bande observée à 797 cm⁻¹ est toujours attribuée à la vibration d'élongation de la liaison Si-C, tandis que les bandes à 882 cm⁻¹ et entre 1000 et 1250 cm⁻¹ (vibrations d'élongation des liaisons Si-O) indiquent une surface partiellement oxydée.

Le spectre FTIR des nanoparticules de silicium poreux greffées avec une porphyrine-NH₂ est présenté sur la figure 13. On observe en particulier deux bandes caractéristiques de la liaison urée à 1620 cm⁻¹ et 1542 cm⁻¹, attribuées respectivement à la vibration d'élongation de la liaison C=O et la la vibration d'élongation de la liaison N-H, ce qui indique le succès du couplage entre les nanoparticules fonctionnalisées avec l'isocyanate et la porphyrine-NH₂.

La quantification de la porphyrine-NH₂ greffée effectuée par dissolution des nanoparticules dans une solution basique de KOH indique que l'on obtient une quantité de 13,3 µg de porphyrine par mg de nanoparticules. Cette quantité est suffisante pour des applications en PDT.

2. Production de ¹O₂ (oxygène) et de ROS (espèces réactives oxydantes) Les mesures consistent à irradier un photosensibilisateur en présence d'une molécule sonde, le diphénylisobenzofurane (DPBF), dont l'oxydation par ¹O₂ se traduit par une diminution du spectre d'absorption. C'est une méthode comparative : en connaissant le rendement quantique de génération de ¹O₂ d'une molécule de référence (comme le rose bengal ou le bleu de méthylène), on en déduit par suivi spectrophotométrique celui des nanoparticules de silicium poreux fonctionnalisées.

Experimental: 2 mL d'une solution de DPBF à 0,08 mM et de nanoparticules (ou de molécule photosensibilisatrice) à concentration connue sont placés dans une cuve en quartz. La cuve est mise sous agitation, fermée et irradiée pendant 15 min. L'irradiation se fait avec une fibre optique de 0,63 cm de diamètre. La lumière est filtrée par un filtre anti-IR pour ne pas chauffer l'échantillon, et un filtre passe-bande entre 410 et 490 nm (figure IV. 1). L'absorbance de la solution est mesurée toutes les minutes pendant les 5 premières minutes, puis toutes les 5 minutes.

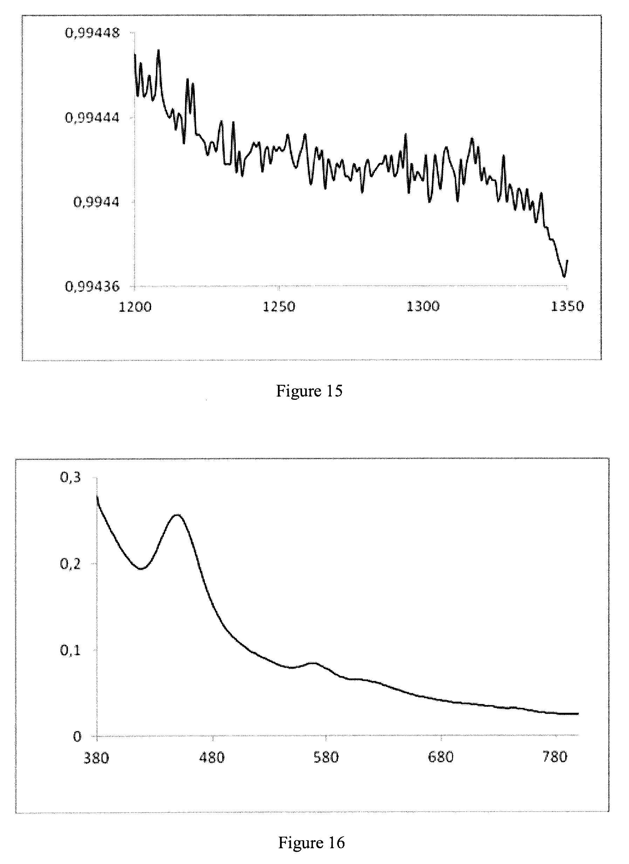

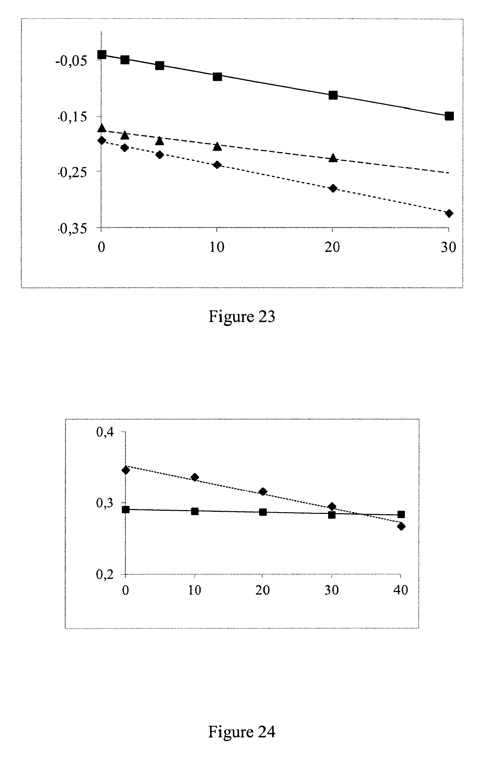

Aucune production d'oxygène singulet n'est observée pour les nanoparticules de Si poreux ne contenant pas de porphyrine (fig. 15). Une production d'oxygène singulet est observée pour les nanoparticules de Si poreux fonctionnalisées avec de la porphyrine-NCS (fig.23), pour les nanoparticules de Si poreux fonctionnalisées avec de la porphyrine-NH₂ (Fig. 24) et pour les nanoparticules de Si poreux fonctionnalisées avec de la porphyrine-NCS du mannose (fig. 17).

### 3. Cinétique de relargage des porphyrines (figures 25, 26)

Expérimental : Le relargage de la porphyrine en fonction du temps a été étudié dans du PBS (phosphate buffer saline) à pH = 7,2, dans du milieu de culture DMEM F12 sans sérum et dans du milieu de culture DMEM F12 à 10% de FBS (Fetal Bovine Serum). Une quantité connue de nanoparticules greffées (environ 500µg) est dispersée dans 3 mL de milieu et mis à incuber à 37°C sur une plaque d'agitation à 100 rpm. A des temps donnés, on prélève 500 µL de chaque dispersion et on rajoute 500 µL du milieu d'étude afin de rester à volume constant. Les 500 µL prélevés sont centrifugés à 22000 g pendant 30 min. Le surnageant est conservé à l'abri de la lumière à 4°C jusqu'à analyse, le culot de nanoparticules est redispersé dans le milieu d'étude.

Les échantillons prélevés sont analysés par spectroscopie d'absorption UV-visible afin de déterminer la concentration en porphyrine de chaque échantillon. La présence du mannose à la surface des nanoparticules de silicium poreux n'influe pas sur leur cinétique de dégradation. Les nanoparticules de silicium poreux sont dégradées totalement après 24 h d'incubation en milieu biologique, elles sont biodégradables.

### 4. Ciblage des tumeurs et " homing"

### 4.1 Imagerie en excitation monophotonique avec les nanoparticules de silicium poreux greffées avec la porphyrine-NH₂ (figure 18)

Experimental : Les cellules MCF-7 sont ensemencées dans des cupules à fond de verre à la densité de 10⁶ cellules/cm². Après 24 heures elles sont rincées une fois puis incubées dans 1 mL de milieu de culture contenant les nanoparticules de silicium poreux à une concentration de 20 µg/mL, et/ou la porphyrine libre à une concentration équivalente pendant 3 ou 5 h selon les expériences. Quinze minutes avant la fin de l'incubation, les membranes des cellules sont marquées avec du Cell Mask Orange (Invitrogen) à une concentration finale de 5 µg/mL (pour l'expérience avec les nanoparticules greffées avec la porphyrine-NH₂ seulement). Elles sont ensuite rincées avec du milieu de culture DMEM blanc (sans rouge de phénol). Les photos de microscopie sont effectuées sur un microscope confocal LSM 780 LIVE. L'excitation de la fluorescence est effectuée à 405 nm ou à 633 nm selon les échantillons. L'émission est récoltée entre 620 et 700 nm.

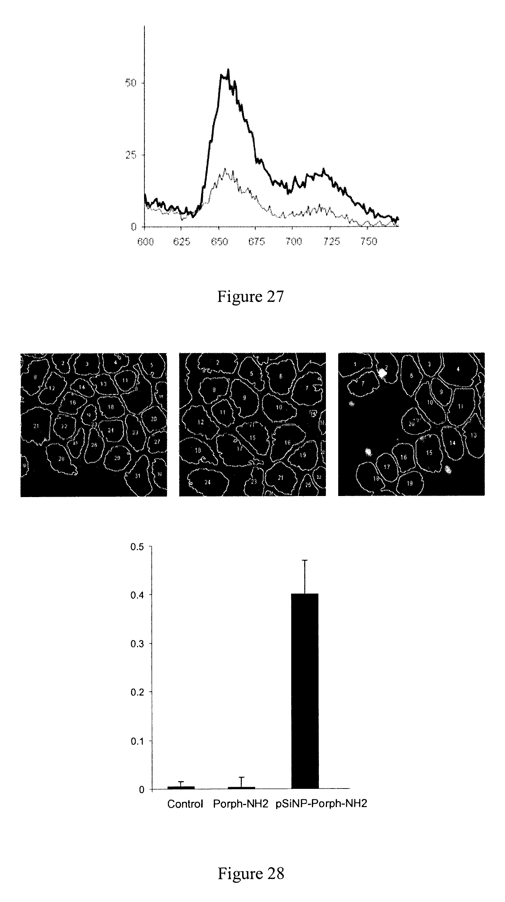

La figure 18 montre également que la porphyrine-NH₂ libre n'est pas détectée à l'intérieur de la cellule, alors que les nanoparticules greffées à la porphyrine-NH₂ sont internalisées et apparaissent luminescentes. Cette expérience montre que l'utilisation de nanoparticules de silicium poreux est un moyen efficace pour internaliser la porphyrine-NH₂ dans les cellules. La quantité de porphyrine internalisées à partir des nanoparticules est dans l'intervalle de 105 ± 5 ng.ml-1 de porphyrines tandis que la porphyrine libre intérnalisée correspond à 35 ± 5 ng.ml-1 (fig. 27 et fig. 28).

### 4.2 Imagerie en excitation monophotonique avec les nanoparticules de silicium poreux greffées avec la porphyrine-NCS (figure 29)

Lorsque les cellules MCF-7 sont incubées avec les nanoparticules de silicium poreux greffées avec la porphyrine-NCS, on observe des points de fluorescence distincts à la surface ou dans les cellules. Ce résultat confirme la possibilité d'internaliser de la porphyrine dans les cellules MCF-7 au moyen de nanoparticules de silicium poreux, et ce même sans agent ciblant. Cette internalisation est possible, par l'action des clathrines et la formation de vésicules d'invagination. Lorsque les cellules MCF-7 sont incubées avec les nanoparticules greffées avec la porphyrine et le mannose, on observe des agrégats de fluorescence intense à l'intérieur des cellules. Les nanoparticules de silicium poreux greffées avec la porphyrine-NCS et le mannose sont donc largement plus présentes que les nanoparticules de silicium poreux greffées avec la porphyrine-NCS sans mannose : le mannose ici joue son rôle d'agent de ciblage avec une grande efficacité. Nous pensons que le mannose à la surface des nanoparticules assure leur endocytose par les cellules.

Des résultats similaires peuvent être obtenus en fonctionnalisant le Si poreux avec des espèces galactose.

### 4.3 Imagerie en excitation biphotonique avec les nanoparticules de silicium poreux greffées avec la porphyrine-NCS (figure 30A et figure 30B)

Experimental: les cellules MCF-7 ont été incubées pendant 5 h avec les différents types de nanoparticules : pSiNP, pSiNP-porphyrine-NCS, pSiNP-mannose, pSiNP-mannose-porphyrine-NCS à 40 µg/mL. Les cellules MCF-7 ont également été incubées pendant 5 h avec de la porphyrine-NCS libre à 3,2 µg/mL, avec des pSiNP et de la porphyrine libre, ainsi qu'avec des pSiNP-mannose et des pSiNP-mannose-porphyrine-NCS avec du mannose libre (à une concentration de 10 mM) afin d'étudier la réversion du ciblage par le mannose. Les cellules ont ensuite été rincées puis imagées après excitation avec un laser pulsé biphotonique à 750 nm. Un colorant membranaire a été ajouté afin de discerner les cellules. La mise au point a été faite en mode confocal.

Lorsque les cellules sont incubées avec les nanoparticules de silicium poreux, on observe quelques zones fluorescentes, indiquant que ces nanoparticules peuvent être internalisées, même si cela semble n'être que dans d'assez faibles proportions. De plus l'observation de la fluorescence ici indique que le silicium poreux peut absorber la lumière en biphotonique.

En comparaison, lorsque les cellules MCF-7 sont incubées avec de la porphyrine-NCS libre, aucune fluorescence n'est observée après excitation biphotonique à 750 nm. Ceci signifie que la porphyrine-NCS présente sur/dans les cellules après rinçage, que l'on pouvait distinguer en imagerie en excitation monophotonique (à 650 nm), n'est pas excitée en lumière biphotonique à 750 nm.

Lorsque les cellules sont incubées avec les nanoparticules de silicium poreux-mannose-porphyrine-NCS, la fluorescence observée est beaucoup plus importante qu'avec les autres types de nanoparticules. La fluorescence est également intracellulaire ce qui indique une internalisation des nanoparticules, qui est nettement améliorée par la présence du mannose à leur surface. Lorsque la même expérience est réalisée en présence d'un excès de mannose libre dans le milieu de culture, on observe un blocage de l'internalisation des nanoparticules qui se caractérise par une fluorescence plus membranaire qu'intracellulaire.

On peut classer la fluorescence observée en excitation biphotonique pour chaque type de nanoparticules, selon l'échelle suivante : porphyrine-NCS libre < pSiNP ∼ pSiNP-mannose ∼ pSiNP + porphyrine-NCS libre < pSinp-porphyrine-NCS < pSiNP-mannose-porphyrine-NCS.

Les résultats obtenus ici montrent que, comme en imagerie monophotonique, la présence du mannose permet une meilleure internalisation des nanoparticules de silicium poreux. La présence combinée de la porphyrine-NCS et du mannose à la surface des nanoparticules de silicium poreux permet d'optimiser leur internalisation dans les cellules et leur visualisation par imagerie biphotonique. Un mécanisme d'action conjointe entre le silicium et la porphyrine-NCS greffée, de type transfert d'énergie, est envisagée pour l'émission de la fluorescence des pSiNP greffées avec la porphyrine.

### 4.4 Efficacité photodynamique en excitation monophotonique

Experimental : Les cellules MCF-7 sont ensemencées dans une plaque de 96 puits, à la densité de 10⁶ cellules/cm². Après 24 h, les cellules sont incubées avec les nanoparticules de silicium poreux à une concentration de 20 µg/mL ou 40 µg/mL pour les nanoparticules respectivement greffées avec la porphyrine-NH₂ et la porphyrine-NCS, ou avec la porphyrine libre à une concentration équivalente pendant 5 h. Les cellules sont alors rincées avec du PBS puis maintenues dans 100 µL de milieu de culture, et enfin irradiées pendant 40 min avec un laser à 650 nm de puissance 7 mW/cm² (soit 16.8 J/cm²). Après 48 h d'incubation, un test MTS (3-(4,5-dimethylthiazol-2-yl)-5-(3-carboxymethoxyphenyl)-2-(4-sulfophenyl)-2*H-*tetrazolium) est effectué afin de quantifier les cellules vivantes dans l'expérience avec les nanoparticules de silicium poreux greffées avec la porphyrine-NCS. Dans le cas des nanoparticules greffées avec la porphyrine-NH₂, un test MTT (bromure de 3-(4,5-diméthylthiazol-2-yl)-2,5-diphényltetrazolium) est effectué afin de quantifier les cellules vivantes. Dans cas du test MTT, les cellules sont incubées pendant 4 h dans du milieu de culture additionné de 0,5 mg/mL de MTT. Le milieu est ensuite écarté et les cristaux violets de MTT sont dissous dans une solution éthanol/DMSO (1:1). L'absorbance de la solution est lue à 540 nm. Dans le cas du test MTS, les cellules sont incubées pendant 2 h dans du milieu de culture additionné de 0,5 mg/mL de MTS. L'absorbance de la solution est alors lue directement à 490 nm.

### 4.4.1 Porphyrine-NH₂ (figure 31)

L'irradiation des cellules cancéreuses seule n'induit aucune toxicité. Les nanoparticules de silicium poreux (pSiNP) ne sont pas cytotoxiques, 99% et 100% des cellules restent vivantes après 5 h d'incubation avec ou sans irradiation respectivement. Le fait que les pSiNP ne soient pas cytotoxiques après irradiation à 650 nm était attendu compte tenu du fait que les pSiNP n'absorbent pas la lumière à cette longueur d'onde.

La porphyrine-NH₂ induit 8% de cytotoxicité en absence d'irradiation. Après irradiation, on observe un taux de mort cellulaire légèrement plus important (16%). Cette cytotoxicité assez faible de la porphyrine libre après irradiation était attendue, puisque les expériences d'imagerie et de quantification montraient que la porphyrine-NH₂ libre était faiblement internalisée par les cellules. En comparaison, les cellules incubées avec les pSiNP-porphyrine-NH₂ et non irradiées présentent 19% de mort cellulaire. Dans ce cas, la toxicité observée à l'obscurité est attribuée à la libération d'une petite quantité de porphyrine-NH₂ cytotoxique à l'intérieur de la cellule, après l'internalisation et la faible métabolisation des nanoparticules. Enfin, les pSiNP-porphyrine-NH₂ induisent 42% de mort cellulaire après irradiation à 650 nm. La toxicité de la porphyrine-NH₂ confinée dans les nanoparticules de silicium poreux est donc significativement augmentée comparée à la porphyrine-NH₂ libre.

### 4.4.2 Porphyrine-NCS (figure 32)

Les pSiNP et les pSiNP-mannose, irradiées à 650 nm, ne sont pas toxiques pour les cellules. 99% et 104% des cellules MCF-7 respectivement survivent après 5 heures d'incubation avec des pSiNP fonctionnalisées ou non au mannose et après irradiation. Dans une expérience parallèle, la toxicité des pSiNP à différentes concentrations sans irradiation a été étudiée sur les mêmes cellules. A 40 µg/mL, les nanoparticules de silicium poreux sont très peu toxiques sans irradiation pour les cellules.

Après incubation de la porphyrine-NCS libre et irradiation, on observe 22 % de mort cellulaire. Suffisamment de porphyrine-NCS se colle aux membranes et/ou est internalisée par les cellules, comme on l'a vu sur les images de microscopie confocale, pour générer un effet non négligeable en PDT monophotonique.

En comparaison, les cellules traitées avec les pSiNP-porphyrine-NCS montrent 28% de mort cellulaire. L'effet de la porphyrine-NCS vectorisée par les nanoparticules ici n'est pas significativement augmenté comparé à l'effet de la porphyrine libre. Par contre lors du traitement par PDT des cellules MCF-7 par les pSiNP-mannose-porphyrine-NCS, le taux de survie cellulaire descend à 58 %. L'accroissement de la cytotoxicité et de l'efficacité de la PDT observé ici est attribué à un ciblage plus efficace des cellules grâce à la présence du mannose, ainsi qu'à une internalisation ou un ancrage à la surface des cellules plus important que pour les pSiNP-porphyrine-NCS, en accord avec les expériences d'imagerie.

### 4.5 Efficacité photodynamique en excitation biphotonique

Une approche particulièrement intéressante consiste à remplacer l'excitation classique à un photon dans le visible par une excitation à deux photons dans le proche infra-rouge, car cela permet de limiter les effets secondaires dus à la lumière dans les tissus traités par thérapie photodynamiques. L'avantage d'une excitation à deux photons est aussi de permettre le traitement de cancers dans des tissus plus profonds.

Un des inventeurs a montré précédemment que les nanoparticules de silicium poreux peuvent être excitées par une excitation en 2-photons dans le proche infra-rouge (780 nm). Ici dans une expérience de PDT à 2-photons, nous montrons que les nanoparticules de Si poreux contenant une porphyrine polycationique excitées en 2-photons (750nm) peuvent tuer des cellules cancéreuses après endocytose des nanoparticules (Figure 19).

Experimental: Les cellules MCF-7 sont ensemencées dans une plaque de 384 puits, à la densité de 10⁶ cellules/cm². Après 24 h, les cellules sont incubées avec les nanoparticules de silicium poreux à une concentration de 40 µg/mL, ou avec la porphyrine libre à une concentration de 3,2 µg/mL pendant 5 h. Les cellules sont alors rincées (cette étape de rinçage n'ayant pas été effectuée pour certaines expériences de contrôle), puis irradiées avec un laser biphotonique à 750 nm. 3 scans de 1,57 s sont effectués. Après 48 h d'incubation, un test MTS est effectué afin de quantifier les cellules vivantes.

Les cellules incubées dans le milieu de culture contrôle seul, et irradiées ne présentent aucune mort cellulaire, ce qui indique que ni le milieu de culture contenant de l'éthanol, ni l'irradiation biphotonique ne sont toxiques pour les cellules. Aucune mort cellulaire n'est non plus observée lorsque les cellules MCF-7 sont incubées avec de la porphyrine-NCS libre, rincées, puis irradiées. Contrairement à ce qui a été observé sous irradiation monophotonique, la porphyrine-NCS restante sur les cellules après rinçage n'est pas sensible à une excitation biphotonique et n'est donc pas toxique pour les cellules.

Lorsque les cellules MCF-7 sont traitées avec les pSiNP non fonctionnalisées, 15 % de mort cellulaire sont observés après rinçage et irradiation biphotonique. Ce résultat indique que les pSiNP, qui sont capables d'absorber la lumière biphotonique, génèrent du ¹O₂ ou d'autres ROS et tuent les cellules. Cependant l'effet observé ici est assez faible. Deux raisons peuvent expliquer ce résultat : il est connu que le rendement quantique de génération de ¹O₂ du silicium poreux est assez faible d'une part ; d'autre part, nous avons observé dans les expériences d'imagerie que l'internalisation des pSiNP n'était pas très importante. En comparaison, on peut observer que les nanoparticules, lorsqu'elles sont fonctionnalisées avec le mannose (pSiNP-mannose) tuent 31 % de cellules après irradiation. La présence de mannose greffé à la surface des nanoparticules semble permettre une meilleure internalisation dans les cellules. Après incubation des cellules avec les pSiNP-porphyrine-NCS, rinçage et irradiation biphotonique, 44 % de mort cellulaire sont observés.

L'hypothèse que nous faisons est basée sur la possibilité d'un transfert d'énergie entre les pSiNP, sensibles à une excitation biphotonique, et la porphyrine-NCS.

Ce résultat suggère un mécanisme de FRET (fluorescence resonance energy transfer) en excitation à 2-photons des nanoparticules de Si poreux aux porphyrines, et par conséquence la génération d'oxygène singulet et d'espèces réactives oxydantes en irradiation à 2-photons.

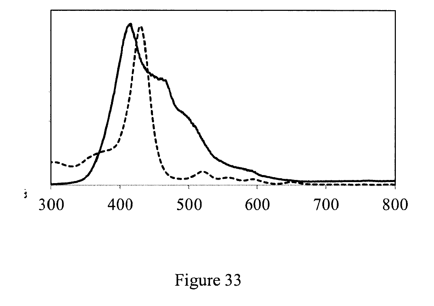

Les conditions nécessaires à un tel transfert, définie par la théorie de Forster-Dexter sont que les deux systèmes donneur et accepteur, le silicium et la porphyrine respectivement dans notre cas, soient suffisamment proches l'un de l'autre dans l'espace (la distance entre l'un et l'autre ne devant pas excéder 10 nm), et que le spectre d'émission du donneur et celui d'absorption de l'accepteur se recouvrent au moins partiellement. Dans notre cas, la proximité du silicium et de la porphyrine est assurée par le greffage covalent. A l'aide du logiciel Chem3D, la distance entre le silicium et la porphyrine a été estimée à 7,8 Å, ce qui est largement inférieur à la distance limite permettant le transfert d'énergie. De plus le recouvrement du spectre d'émission du donneur et de celui d'absorption de l'accepteur est démontré sur la figure 33.

Enfin, lorsque les cellules MCF-7 sont incubées avec les pSiNP-mannose-porphyrine-NCS, le taux de mort cellulaire observé est de 30 %. Ce résultat est surprenant. En effet on s'attendait à avoir un taux de mort cellulaire maximal pour ces nanoparticules puisque d'une part, la quantité de porphyrine greffée est plus importante que sur les nanoparticules greffées seulement avec la porphyrine, et que d'autre part la présence du mannose-squarate à la surface des nanoparticules améliore l'internalisation des nanoparticules par les cellules. Or ces nanoparticules ont un effet moindre en PDT à 2 photons que les nanoparticules greffées avec la porphyrine. Considérant l'hypothèse du transfert d'énergie entre les pSiNP et la porphyrine-NCS, ceci est expliqué par l'absorption par le mannose d'une partie de l'énergie transmise. En effet le mannose, pour être greffé à la surface des pSiNP, possède un groupe phényl et un groupe squarate. L'aromaticité et la conjugaison du squarate sont soupçonnées d'être à l'origine d'une désactivation de l'état excité du silicium poreux, et explique cette absorption de l'énergie. Un autre type de lien pour le mannose, telle qu'une cétone, décrit précédemment peut être utilisé pour le greffage du mannose.

## Revendications

1. Nanoparticules répondant à la formule (I) : 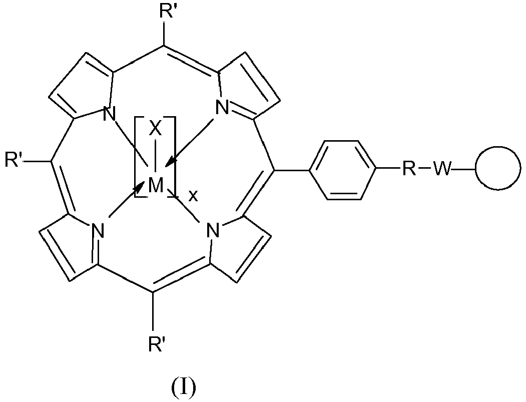 dans laquelle : 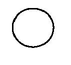
représente une nanoparticule de silicium poreux,
x représente un entier choisi parmi : 0 et 1,
M représente un atome de métal choisi parmi les métaux de transition,
X représente un groupement choisi parmi : un halogénure, un anion d'un acide carboxylique pharmaceutiquement acceptable,
R représente un groupement choisi parmi : une urée (-NH-CO-NH-), une thiourée (-NH-CS-NH-),
W représente un groupement alcane di-yle en C1-C12,
R' représente un groupement choisi parmi : 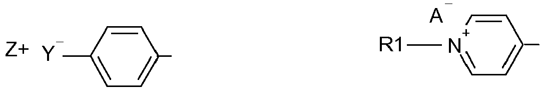
Z⁺ représente un cation organique ou minéral pharmaceutiquement acceptable,
Y⁻ représente un groupement qui peut être choisi parmi : -COO⁻, -SO₃⁻
A⁻ représente un anion qui peut être choisi parmi : un halogénure, un anion d'un acide carboxylique pharmaceutiquement acceptable,
R1 représente un alkyle en C1 à C10.

2. Nanoparticules selon la revendication 1 répondant à la formule (Ia) : 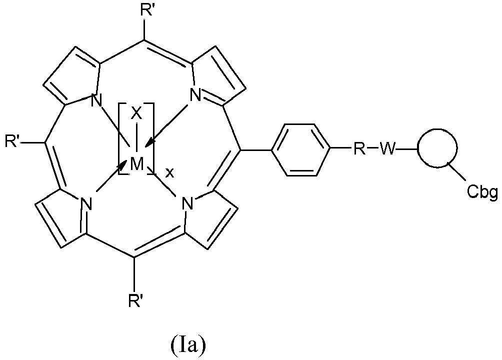 Dans laquelle Cbg représente une molécule de ciblage spécifique des tissus néoplasiques.

3. Nanoparticules selon la revendication 1 ou la revendication 2 dont la taille est de 20 à 200 nm, avantageusement de 50 à 190 nm, encore mieux, de 100 à 175 nm, et de préférence de 140 à 160 nm et la taille des pores allant de 5 à 50 nm, avantageusement de 10 à 30 nm.

4. Nanoparticules selon l'une quelconque des revendications 1 à 3, dans lesquelles x représente 0.

5. Nanoparticules selon l'une quelconque des revendications 1 à 4, dans lesquelles :
X représente un groupement choisi parmi : Cl⁻, Br⁻, I⁻, acétate, propionate, butyrate, ascorbate, benzoate, cinnamate, citrate, fumarate, glycolate, malonate, tartrate, malate, maléate, mandélate, tosylate,
W représente un groupement -(CH₂)₃-.
R1 représente un groupement choisi parmi : un alkyle en C1-C3,
Z⁺ représente un cation qui peut être choisi parmi : K⁺, Li⁺, Na⁺, NH₄⁺,
A représente un anion qui peut être choisi parmi : Cl⁻, Br⁻, I⁻, acétate, propionate, butyrate, ascorbate, benzoate, cinnamate, citrate, fumarate, glycolate, malonate, tartrate, malate, maléate, mandélate, tosylate,
R' est choisi parmi : 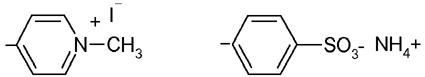 Cbg, lorsqu'il est présent, est choisi parmi les sucres et les dérivés de sucres.

6. Nanoparticules selon la revendication 5 choisies parmi :

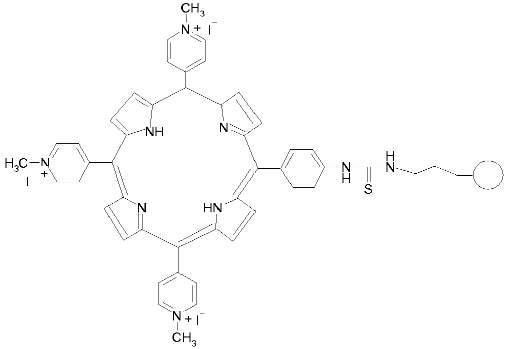

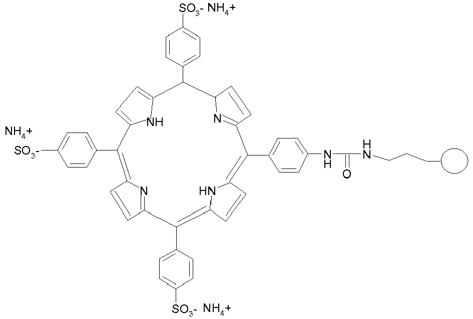

7. Procédé de fabrication de nanoparticules selon l'une quelconque des revendications 1 à 6, ce procédé comprenant les étapes de :
(i) fourniture de nanoparticules de silicium poreux,
(ii) fonctionnalisation des nanoparticules de silicium poreux par des groupements comportant au moins une fonction NH₂ ou au moins une fonction isocyanate, isothiocyanate ou semicarbazide,
(iii) fourniture et greffage d'une molécule photosensibilisante, porteuse d'un groupement complémentaire de celui porté par la nanoparticule, de façon à former une liaison urée ou thiourée, et éventuellement
(iv) greffage par au moins une molécule de ciblage.

8. Procédé selon la revendication 7, qui comporte les étapes de :
(i)
a- gravure électrochimique de plaquettes de silicium monocristallin dans une solution éthanolique d'acide fluorhydrique (HF),
b- élimination du film poreux et traitement par ultrasons,
(ii)
a- oxydation ménagée suivie d'une silanisation de façon à produire des groupements Si-OH et des espèces SiO₂ à la surface des nanoparticules de silicium poreux, et/ou
b- hydrosilylation,
(iii) greffage des nanoparticules par une molécule photosensibilisante de type porphyrine répondant à la formule (II) dans laquelle Q représente un groupement choisi parmi : -NH₂, -N=C=O, -N=C=S, 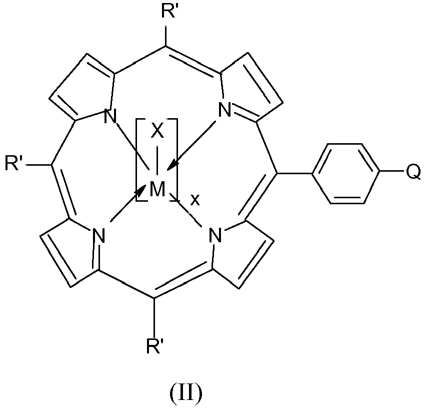
(iv) greffage des nanoparticules par un sucre ou un dérivé de sucre à l'aide d'un groupement phénylsquarate ou d'un groupement semicarbazone.

9. Nanoparticules selon l'une quelconque des revendications 1 à 6, pour leur utilisation pour la détection, le traitement, le suivi, la prévention, pour retarder l'apparition et/ou la récidive d'une pathologie choisie parmi les cancers, les tumeurs, les maladies prolifératives cellulaires, les maladies de la peau.

10. Composition de médicament comprenant des nanoparticules selon l'une quelconque des revendications 1 à 6 dans un support pharmaceutiquement acceptable.

11. Composition cosmétique comprenant des nanoparticules selon l'une quelconque des revendications 1 à 6 dans un support cosmétiquement acceptable

12. Kit pour la détection, le traitement, le suivi, la prévention, pour retarder l'apparition et/ou la récidive d'une pathologie choisie parmi les cancers, les tumeurs, les maladies prolifératives cellulaires comprenant :
- des nanoparticules de silicium poreux greffées par l'intermédiaire d'une liaison covalente par au moins une molécule photosensibilisante, ou une composition de médicament les comprenant
et
- des moyens permettant une irradiation biphotonique dans l'infra-rouge.

13. Kit pour la détection, le traitement, le suivi, la prévention, pour retarder l'apparition et/ou la récidive d'une pathologie choisie parmi les cancers, les tumeurs, les maladies prolifératives cellulaires comprenant :
- des nanoparticules selon l'une quelconque des revendications 1 à 6, ou une composition selon la revendication 10,
et
- des moyens permettant une irradiation dans l'infra-rouge.

## Patentansprüche

1. Nanopartikel mit der Formel (I): 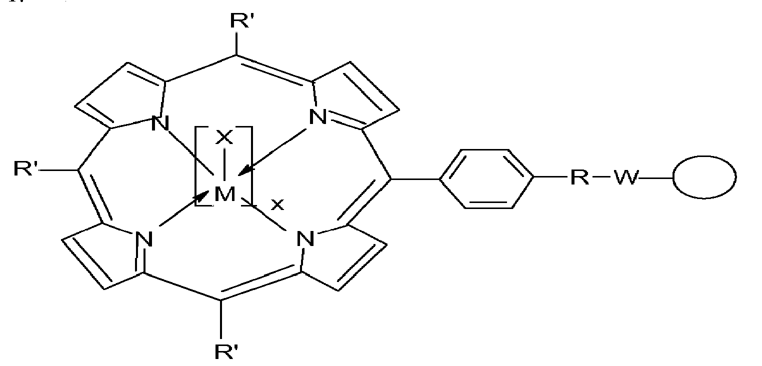 wobei: 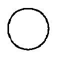 für einen porösen Siliciumnanopartikel steht,
- x für eine ganze Zahl steht, ausgewählt aus: 0 und 1,
- M für ein Metallatom steht, ausgewählt aus den Übergangsmetallen,
- X für eine Gruppe steht, ausgewählt aus: einem Halogenid, einem Anion einer pharmazeutisch verträglichen Carbonsäure,
- R für eine Gruppe steht, ausgewählt aus: einem Harnstoff (-NH-CO-NH-), einem Thioharnstoff (-NH-CS-NH),
- W für eine (C₁-C₁₂)-Alkandiylgruppe steht,
- R' für eine Gruppe steht, ausgewählt aus: 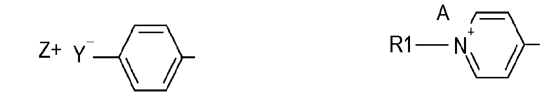
- Z⁺ für ein pharmazeutisch verträgliches organisches oder anorganisches Kation steht,
- Y⁻ für eine Gruppe steht, die ausgewählt sein kann aus: -COO⁻, -SO₃⁻
- A⁻ für ein Anion steht, das ausgewählt sein kann aus: einem Halogenid, einem Anion einer pharmazeutisch verträglichen Carbonsäure,
- R1 für ein (C₁ bis C₁₀)-Alkyl steht.

2. Nanopartikel nach Anspruch 1 mit der Formel (Ia): 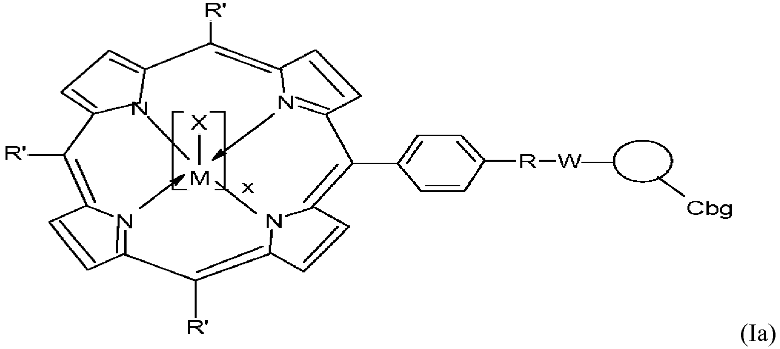 wobei Cbg für ein Molekül zum spezifischen Targeting von neoplastischen Geweben steht.

3. Nanopartikel nach Anspruch 1 oder Anspruch 2, deren Größe 20 bis 200 nm, vorteilhafterweise 50 bis 190 nm, noch besser 100 bis 175 nm und bevorzugt 140 bis 160 nm beträgt und die Porengröße liegt im Bereich von 5 bis 50 nm, vorteilhafterweise von 10 bis 30 nm.

4. Nanopartikel nach einem der Ansprüche 1 bis 3, wobei x für 0 steht.

5. Nanopartikel nach einem der Ansprüche 1 bis 4, wobei:
- X für eine Gruppe steht, ausgewählt aus: Cl⁻, Br⁻, I⁻, Acetat, Propionat, Butyrat, Ascorbat, Benzoat, Cinnamat, Citrat, Fumarat, Glycolat, Malonat, Tartrat, Malat, Maleat, Mandelat, Tosylat,
- W für eine Gruppe -(CH₂)₃₋ steht.
- R1 für eine Gruppe steht, ausgewählt aus einem (C₁-C₃)-Alkyl,
- Z⁺ für ein Kation steht, das ausgewählt sein kann aus: K⁺, Li⁺, Na⁺, NH₄⁺,
- A für ein Anion steht, das ausgewählt sein kann aus: Cl, Br⁻, I⁻, Acetat, Propionat, Butyrat, Ascorbat, Benzoat, Cinnamat, Citrat, Fumarat, Glycolat, Malonat, Tartrat, Malat, Maleat, Mandelat, Tosylat,
- R' ausgewählt ist aus: 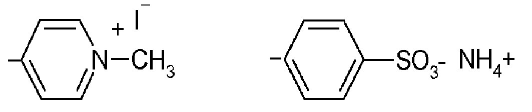 Cbg, sofern vorhanden, ausgewählt ist aus Zuckern und Zuckerderivaten.

6. Nanopartikel nach Anspruch 5, ausgewählt aus:

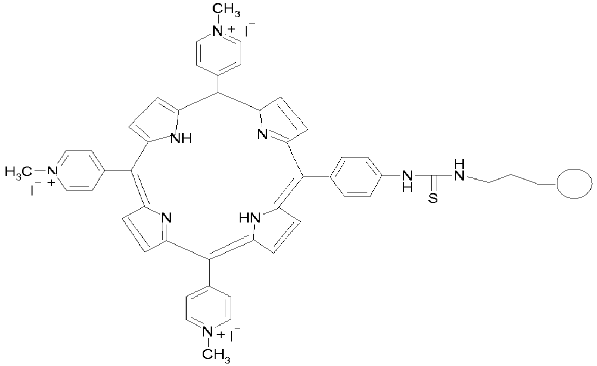

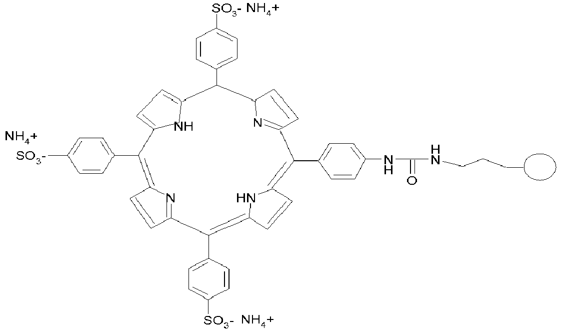

7. Verfahren zur Herstellung von Nanopartikeln nach einem der Ansprüche 1 bis 6, wobei dieses Verfahren die folgenden Schritte umfasst:
(i) Bereitstellen von porösen Siliciumnanopartikeln,
(ii) Funktionalisieren der porösen Siliciumnanopartikel mit Gruppen, die mindestens eine NH₂-Funktion oder mindestens eine Isocyanat-, Isothiocyanat- oder Semicarbazidfunktion umfassen,
(iii) Bereitstellen und Pfropfen eines photosensi-bilisierenden Moleküls, das eine Gruppe trägt, die komplementär ist zu der, die von dem Nanopartikel getragen wird, um eine Harnstoffbindung oder Thioharnstoffbindung zu bilden, und gegebenenfalls
(iv) Pfropfen mit mindestens einem Targeting-Molekül.

8. Verfahren nach Anspruch 7, das die folgenden Schritte umfasst:
(i)
a- elektrochemisches Ätzen von monokristallinen Siliciumplatten in einer Fluorwasserstoff-säure(HF)-Ethanollösung,
b- Entfernen des porösen Films und Behandeln mit Ultraschall,
(ii)
a- kontrolliertes Oxidieren, gefolgt von einer Silanisierung, um Si-OH-Gruppen und SiO₂-Spezies an der Oberfläche der porösen Siliciumnanopartikel zu erzeugen, und/oder
b- Hydrosilylierung,
(iii) Pfropfen der Nanopartikel mit einem photo sensibilisierenden Molekül vom Typ Porphyrin mit der Formel (II), wobei Q für eine Gruppe steht, ausgewählt aus: -NH₂, -N=C=O, -N=C=S, 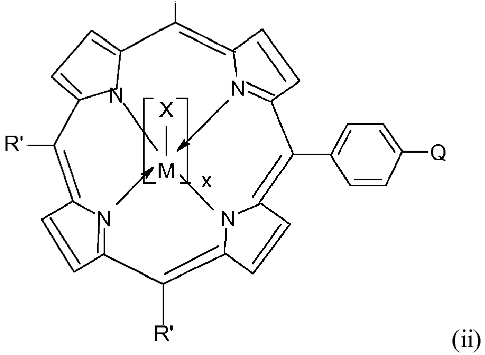
(iv) Pfropfen der Nanopartikel mit einem Zucker oder einem Zuckerderivat mithilfe einer Phenylsquaratgruppe oder einer Semicarbazongruppe.

9. Nanopartikel nach einem der Ansprüche 1 bis 6 zur Verwendung davon beim Nachweis, bei der Behandlung, der Überwachung, der Vorbeugung, zum Verzögern des Auftretens und/oder des Rezidivierens einer Erkrankung, ausgewählt aus Krebsarten, Tumoren, Zellproliferationskrankheiten, Haut-krankheiten.

10. Arzneimittelzusammensetzung, umfassend Nanopartikel nach einem der Ansprüche 1 bis 6 in einem pharmazeutisch verträglichen Träger.

11. Arzneimittelzusammensetzung, umfassend Nanopartikel nach einem der Ansprüche 1 bis 6 in einem kosmetisch verträglichen Träger.

12. Kit zum Nachweis, zur Behandlung, zur Überwachung, zur Vorbeugung, zum Verzögern des Auftretens und/oder des Rezidivs einer Erkrankung, ausgewählt aus Krebsformen, Tumoren, Zellproliferationskrankheiten, umfassend:
- poröse Siliciumnanopartikel, die mittels einer kovalenten Bindung mit mindestens einem photo-sensibilisierenden Molekül gepfropft sind, oder eine Arzneimittelzusammensetzung, die diese umfasst,
und
- Einrichtungen, die eine Zwei-Photonen-Bestrahlung im Infrarotbereich ermöglichen.

13. Kit zum Nachweis, zur Behandlung, zur Überwachung, zur Vorbeugung, zum Verzögern des Auftretens und/oder des Rezidivs einer Erkrankung, ausgewählt aus Krebsformen, Tumoren, Zellproliferationskrankheiten, umfassend:
- Nanopartikeln nach einem der Ansprüche 1 bis 6, oder eine Zusammensetzung nach Anspruch 10, und
- Einrichtungen, die eine Bestrahlung im Infrarotbereich ermöglichen.

## Claims

1. Nanoparticles corresponding to formula (I): 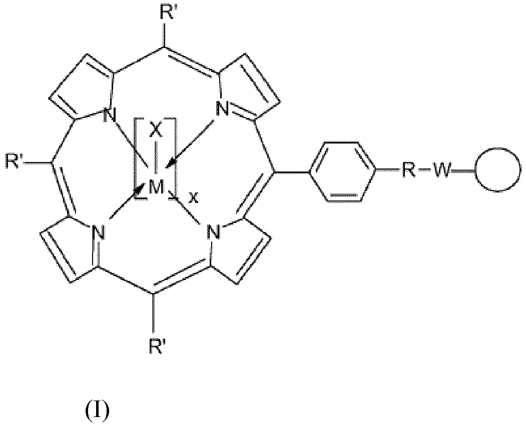 in which: 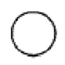 represents a porous silicon nanoparticle,
x represents an integer chosen from: 0 and 1,
M represents a metal atom chosen from the transition metals,
X represents a group chosen from: a halide, an anion of a pharmaceutically acceptable carboxylic acid,
R represents a group chosen from: a urea (-NH-CO-NH-), a thiourea (-NH-CS-NH-),
W represents a C1-C12 alkanediyl group,
R' represents a group chosen from: 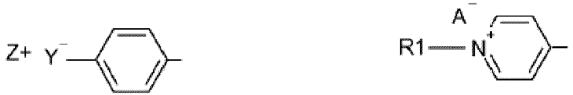
Z⁺ represents a pharmaceutically acceptable organic or mineral cation.
Y⁻ represents a group which can be chosen from: -COO⁻, -SO₃⁻
A⁻ represents an anion which can be chosen from: a halide, an anion of a pharmaceutically acceptable carboxylic acid,
R1 represents a C1 to C10 alkyl.

2. Nanoparticles according to claim 1 corresponding to formula (Ia): 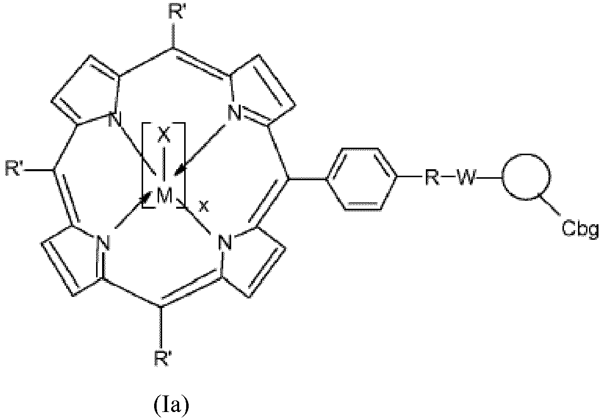 In which Cbg represents a specific targeting molecule for neoplastic tissues.

3. Nanoparticles according to claim 1 or claim 2, the size of which is from 20 to 200 nm, advantageously from 50 to 190 nm, better still from 100 to 175 nm, and preferably from 140 to 160 nm, and the pore size ranges from 5 to 50 nm, advantageously from 10 to 30 nm.

4. Nanoparticles according to any one of claims 1 to 3, in which x represents 0.

5. Nanoparticles according to any one of claims 1 to 4, in which:
X represents a group chosen from: Cl⁻, Br⁻, I⁻, acetate, propionate, butyrate, ascorbate, benzoate, cinnamate, citrate, fumarate, glycolate, malonate, tartrate, malate, maleate, mandelate, tosylate,
W represents a -(CH₂)₃- group.
R1 represents a group chosen from: a C1-C3 alkyl,
Z⁺ represents a cation which can be chosen from: K⁺, Li⁺, Na⁺, NH₄⁺,
A represents an anion which can be chosen from: Cl⁻, Br⁻, I⁻, acetate, propionate, butyrate, ascorbate, benzoate, cinnamate, citrate, fumarate, glycolate, malonate, tartrate, malate, maleate, mandelate, tosylate,
R' is chosen from: 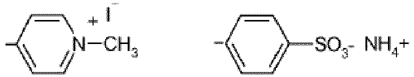
Cbg, if present, is chosen from sugars and derivatives of sugars.

6. Nanoparticles according to claim 5, chosen from:

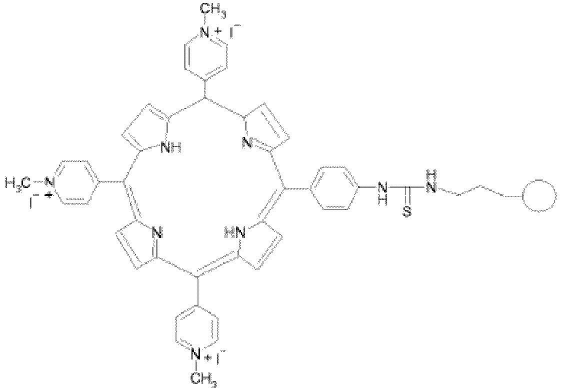

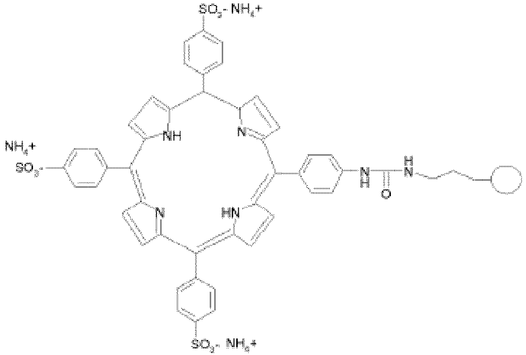

7. Method for producing nanoparticles according to any one of claims 1 to 6, this method comprising the steps of:
(i) providing porous silicon nanoparticles,
(ii) functionalizing the porous silicon nanoparticles with groups comprising at least one NH₂ function or at least one isocyanate, isothiocyanate or semicarbazide function,
(iii) providing and grafting a photosensitizing molecule, which bears a group complementary to the one borne by the nanoparticle, so as to form a urea or thiourea bond, and optionally
(iv) grafting with at least one targeting molecule.

8. Method according to claim 7, which comprises the steps of:
(i)
a- electrochemical etching of monocrystalline silicon plates in a hydrofluoric acid (HF) ethanol solution,
b- removal of the porous film and treatment by ultrasound,
(ii)
a- controlled oxidation followed by silanization so as to produce Si-OH groups and SiO₂ species on the surface of the porous silicon nanoparticles, and/or
b- hydrosilylation,
(iii) grafting the nanoparticles with a porphyrin-type photosensitizing molecule corresponding to formula (II), in which Q represents a group chosen from: -NH₂,
- N=C=O, -N=C=S, 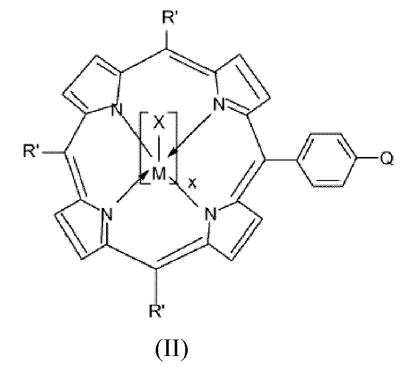
(iv) grafting the nanoparticles with a sugar or a sugar derivative, with the aid of a phenylsquarate group or a semicarbazone group.

9. Nanoparticles according to any one of claims 1 to 6, for the use thereof to detect, treat, monitor, prevent, delay the appearance and/or recurrence of a pathology chosen from cancers, tumours, cell proliferation diseases, skin diseases.

10. Medicinal composition comprising nanoparticles according to any one of claims 1 to 6 in a pharmaceutically acceptable support.

11. Cosmetic composition comprising nanoparticles according to any one of claims 1 to 6 in a cosmetically acceptable support.

12. Kit for the detection, treatment, monitoring, prevention, delay of the appearance and/or recurrence of a pathology chosen from cancers, tumours, cell proliferation diseases, comprising:
- porous silicon nanoparticles grafted via a covalent bond with at least one photosensitizing molecule, or a medicinal composition comprising them
and
- means that allow a biphotonic irradiation in the infrared.

13. Kit for the detection, treatment, monitoring, prevention, delay of the appearance and/or recurrence of a pathology chosen from cancers, tumours, cell proliferation diseases, comprising:
- nanoparticles according to any one of claims 1 to 6, or a composition according to claim 10,
and
- means that allow an irradiation in the infrared.
